# EUROPÄISCHE PATENTSCHRIFT

(11) **EP 1 660 023 B1**
(45) Veröffentlichungstag und Bekanntmachung des Hinweises auf die Patenterteilung: **07.01.2009**
(21) Anmeldenummer: 04764537.9
(22) Anmeldetag: 27.08.2004
(51) Int. Cl.: A61K 8/18, A61K 8/99, A61Q 19/00, A61Q 5/00

(54) **KAPSEL DEREN KAPSELHÜLLE BEI TOPISCHER ANWENDUNG NICHT MEHR GESONDERT WAHRNEHMBAR IST**
CAPSULE WHOSE ENVELOPE IS SEPARATELY IMPERCEPTIBLE DURING THE TOPICAL USE THEREOF
CAPSULE DONT L'ENVELOPPE EST SEPAREMENT IMPERCEPTIBLE PENDANT L'APPLICATION TOPIQUE

(30) Priorität: 27.08.2003 DE 10339747; 30.08.2003 DE 10340106; 10.12.2003 DE 10357639
(43) Veröffentlichungstag der Anmeldung: 31.05.2006
(60) Teilanmeldung: 08016946.9
(73) Patentinhaber: Beiersdorf AG, 20253 Hamburg (DE)
(72) Erfinder: KALLMAYER, Volker, 22393 Hamburg (DE); NIELSEN, Jens, 24558 Henstedt-Ulzburg (DE); BLECKMANN, Andreas, 22926 Ahrensburg (DE); RIEDEL, Heidi, 22083 Hamburg (DE); ECKERT, Julia, 20257 Hamburg (DE); RASCHKE, Thomas, 25421 Pinneberg (DE); TEUBER, Frank, 22846 Norderstedt (DE); ZU PUTLITZ, Corinna, 22339 Hamburg (DE); CONZELMANN, Stephanie, 21149 Hamburg (DE); LISTE, Kathrin, 22301 Hamburg (DE); KRUSE, Uta, 20149 Hamburg (DE); CIERPISZ, Yvonne, 20251 Hamburg (DE)
(86) Internationale Anmeldenummer: PCT/EP2004/009563
(87) Internationale Veröffentlichungsnummer: WO 2005/020940

(56) Entgegenhaltungen:
- EP-A- 0 234 078
- EP-A- 1 129 771
- EP-A- 1 201 219
- WO-A-01/03538
- WO-A-01/38174
- DE-A- 4 223 004
- DE-A- 10 209 111
- US-A1- 2002 022 038
- DATABASE WPI Section Ch, Week 200355 Derwent Publications Ltd., London, GB; Class A14, AN 2003-580900 XP002313041 & JP 2003 073230 A (SHISEIDO CO LTD) 12. März 2003 (2003-03-12)

## Beschreibung

Die Erfindung umfasst kosmetische und dermatologische Zubereitungen in Form von Kapseln umfassend eine Kapselhülle, die fest, halbfest und/oder formstabil ist und im Wesentlichen aus Wachsen, Emulgatoren, natürlichen und/oder synthetischen Polymeren und/oder Mischungen daraus besteht, sowie das Verfahren zu ihrer Herstellung. Des Weiteren umfasst die Erfindung eine Kapsel mit einer Füllung bestehend aus einem oder mehreren festen, halbfesten, pastösen und/oder flüssigen Inhaltsstoffen. Optional kann die Hülle zusätzlich Wasser und/oder Polyole enthalten.

Die Eigenschaft der Hülle ist charakterisiert dadurch, dass sie
- beim Verreiben und/oder Verteilen der Zubereitung auf der Haut und/oder dem Haar schmilzt und/oder aufgrund von Scherkräften völlig oder teilweise flüssig wird
- und/oder sie sich in der Füllung und/oder den Hautsebumlipiden bzw. durch Vermischung von innerer Phase und Hüllmaterial auflöst
und so für den Anwender nicht mehr als.gesondert neben der Füllung vorliegender Bestandteil der Zubereitung wahrnehmbar ist.

Aus dem Stand der Technik sind pharmazeutische Kapseln, beispielsweise Weich- oder Hartgelantinekapseln bekannt, die aus einer Hülle aus Gelantine und Glycerol sowie gelegentlich Farbstoffen, und einer flüssigen, pastösen oder partikulärer Füllung bestehen. Das Verfahren zur Herstellung dieser seit vielen Jahren bekannten Kapseln erfolgt z.B. nach dem sog. Rotary-Die-System (Seifen-Öle-Fette-Wachse, 113. Jg; Nr 311987, Seite 67 ff.). Diese GelantinekapseIn lösen sich beim Verschlucken im Magen-Darmtrakt auf und geben ihre Inhaltstoffe frei. Da es ohne weiteres möglich ist, Kapseln in unterschiedlichen Größen und Formen zu erzeugen, ist die Anwendung nicht nur auf auf perorale Applikationsformen beschränkt (Rudolf Voigt, "Pharmazeutische Technologie", Deutsche Apotheker Verlag, Stuttgart, 9. Auflage (2000), S.543ff). Eine orale Resorption kann durch Lutschkapseln erzielt werden. Sie sind innen hohl und besitzen eine dreifach stärkere Wanddicke als andere Kapseln. Der Wirkstoff ist hierbei in der Gelantinehülle eingearbeitet. Ein weiteres Beispiel sind Nitroglycerin-Kaukapseln (Zerbeißkapseln), die ebenfalls eine rasche Resorption des Wirkstoffs über die Mundschleimhaut ermöglichen. Des Weiteren können einzeldosierte Arzneistoffe nach Aufstechen oder Aufschneiden der tubenförmigen Salbenkapseln durch Ausquetschen des Inhalts zur Applikation gebracht werden (perkutane Applikation von Nitroglycerin-Herzsalbe).

Aus dem Lebensmittelsbereich sind beispielsweise Pralinees bekannt, die in einer formstabilen Schokoladenhülle flüssige oder pastöse Inhaltsstoffe enthalten. Die Hülle schmilzt beim Lutschen oder Zerbeißen im Mund oder nach dem Verschlucken.

Im Kosmetikbereich sind die sogenannten Badeperlen bekannt, deren Hülle, z.B aus Gelatine sich im heißen oder warmen Badewasser rückstandsfrei auflöst und ihren Inhalt, beispielsweise Tensidzubereitungen, Emulsionen, Lipide, Farbstoffe und/oder Parfume, ins Badewasser freigibt. Da die Hülle aus Gelatine besteht, darf das Produkt kein Wasser enthalten, andernfalls würde die Hülle während der Lagerung aufweichen.

Eine zweite Gruppe kosmetischer Kapseln umfasst alle Produkte, für welche die Kapselhülle nur ein Behältnis zur einmaligen Dosierung und Anwendung darstellt und deren Hülle nach der Anwendung übrig bleibt. Nachteilig ist hieran, dass die übrig bleibende Hülle störend wirkt und zudem entsorgt werden muss.

Zahlreiche kosmetische und/oder dermatologische Wirkstoffe sind gegenüber bestimmten Einflüssen wie Feuchtigkeit, niedrigen oder hohen pH-Werten sowie Sauerstoff oder Licht nicht stabil. Es hat daher nicht an Versuchen gefehlt, derartige Wirkstoffe den genannten unerwünschten Umwelteinflüssen so zu entziehen, dass dennoch bei der Anwendung eine Freisetzung der Wirkstoffe stattfindet. Ein Weg zur Erreichung dieses Ziels ist die Mikro- oder Nanoverkapselung von Wirkstoffen. Das Verkapselungsmaterial als Trägersystem für die Wirkstoffe erlaubt dabei, sie vor Umwelteinflüssen geschützt in geeignete Zubereitungen einzuarbeiten, ohne dass der Verwender die Kapseln bei der Produktapplikation wahrnehmen könnte.

Ziel einer solchen Verkapselung ist es beispielsweise, wirkstoffhaltige Wachspartikel im Mikrometerbereich (1 - 250 µm) herzustellen, die in gängigen pastösen oder flüssigen kosmetischen Zubereitungen einzuarbeiten sind. Es ist bisher nicht bekannt, diese Mikrokapseln als eigenständige kosmetische Zubereitung herzustellen, zu lagern und topisch anzuwenden. In der DE 10210449 werden wachsbeschichtete Kapseln, die mit Wirkstoff beladen sind, offenbart. Diese Kapseln werden mittels eines so genannten Wirbelschichtverfahrens hergestellt, so dass maximal Kapseln mit einer Größe von 200 µm hergestellt werden können.

Zur Verkapselung kosmetischer Wirkstoffe gibt es mehrere Ansätze. Bekannt ist beispielsweise die liposomale Verkapselung von Arzneistoffen, die zu einer retardierten Wirkstofffreisetzung führen soll. Im Wesentlichen handelt es sich dabei um von Phospholipiden oder anderen Amphiphilen umschlossene wirkstoffhaltige sphärische Vesikel, die sogenannten Liposome. Die Langzeitstabilität derartiger Gebilde ist jedoch gering. Nanopartikel sind feste Partikel mit Partikelgrößen von 20 bis 500 nm. Gelegentlich werden auch größere Teilchen mit Durchmessern bis zu 1000 nm zu den Nanopartikeln gerechnet. Derartige Partikel bestehen in der Regel aus Polymeren und weisen Kavitäten auf oder bilden eine Hülle, so dass sich in ihrem Inneren Gastmoleküle aufhalten können, die umschlossen oder adsorbiert werden. Diese Gastmoleküle werden dann bei der Anwendung des Nanopartikel enthaltenden Produktes langsam freigesetzt. Ähnlich verhalten sich feste Lipidnanopartikel, die in einer Matrix aus festen Lipiden verteilte Wirkstoffe enthalten. Die Größe der Partikel ist mit denen der Nanopartikel vergleichbar.

Zur Verkapselung von pharmazeutischen oder kosmetischen Wirkstoffen zur Kontrolle der Wirkstofffreisetzung oder der stabilen Einarbeitung in Zubereitungen sind zahlreiche Methoden bekannt.

Die europäische Patentanmeldung EP 1064911 bzw. EP 1064912 offenbart Wirkstoff enthaltende Mikrokapseln mit einem Durchmesser von 0,1 bis 5 mm, die man erhält, in dem man aus Gelbildnern, Chitosan und Wirkstoff eine Matrix herstellt, und diese in wässrige Lösungen anionischer Polymere eintropft. Dabei bildet sich aus Chitosan und anionischem Polymer eine Membran, die die Wirkstofflösung umhüllt. Diese Mikropartikel werden dann wiederum als Bestandteil gängiger kosmetischer Zubereitungen verwendet. Allgemeines zu Verkapselungstechniken mit Chitosan findet sich in Journal of Microencapsulation 14, Seiten 689-711 (1997).

In Lipidpartikein verkapselte, zumeist lipophile Wirkstoffe sind an sich dem Fachmann bekannt. So beschreiben die EP 167825, DE 100 59 668, DE 199 45 203, EP 0934743, WO 94/20072, WO 00/10522 sowie die WO 00/67728 wirkstoffbeladene Lipidpartikel. Doch konnten diese Schriften nicht das Problem der Bereitstellung von kapselförmigen Zubereitungen mit umschlossenen flüssigen, pastösen oder festen kosmetischen Inhaltsstoffen lösen, die als eigenständige kosmetische Zubereitung herzustellen, zu lagern und topisch anzuwenden sind.

WO 01/03538 A1 offenbart verkapselte Zubereitungen, deren Kapselhollen aus einem Polymer bestehen und eine Größe von bis zu 5 mm aufweisen. Die Kapseln müssen für eine manuelle Entnahme durch eine Stoffschicht "ausgedrückt" und appliziert werden.

US 20021022038 A1 offenbart Mikrokapseln mit einem Durchmesser von max. 1000 µm, bevorzugt 15 - 25 µm. Die Kapselhüllen bestehen aus einem Gemisch aus Wachsen und Polymeren.

EP 234078 A1 beschreibt Mikrokapseln mit einer Größe bis 5 mm. Die wasserfreie Kapselhülle besteht aus Wachsen und Polymeren, wie Eudragit ®.

EP 1129771 A1 beschreibt Mikrokapseln mit einer Kapselgröße bis 5 mm und einer Hülle aus wässrigen Gemischen von Ölkörpem, Emulgatoren und Polymeren, insbesondere aus Chitosan.

JP 2003073230 A1 (Abstrakt) beschreibt kosmetische Kapseln, die sich in einer polymerhaltigen wässrigen Lösung nach Zuführung einer geschmolzenen wachsartigen Mischung nach anschließender Abkühlung ausbilden.

DE 4223004 A1 beschreibt einzeldosierte topische Zubereitung in Form von in Blistern verpackten Weichgelatinekapseln, in denen eine Füllung aus ein oder mehreren festen, halbfesten, pastösen und/oder flüssigen kosmetische und/oder dermatologische Inhaltsstoffen enthalten sind, die eine direkte Anwendung auf der Haut ermöglichen.

DE 10209111 A1 offenbart Tücher, die Produktvesikel enthalten, deren Kapselhülle aus Polymeren gebildet wird.

Aus DE 19852262, DE 9321186 und CH 692968 sind Verfahren zur Herstellung von Süssmassen, insbesondere Schokoladenartikeln, nach dem sog. one-shot, frozen-cone bzw. cold-stamp Verfahren bekannt.

Die hierin beschriebenen Verfahren befassen sich ausschließlich mit der Herstellung von Schokoladenartikeln, Pralinés.

Aufgabe der vorliegenden Erfindung ist es, ein Verfahren zur Herstellung kosmetischer Zubereitung bereit zu stellen, die als feste, halbfeste bzw. formstabile Kapsel vorliegt, damit einzeln portionierbar ist und als Ganzes auf der Haut verteilt werden kann. Insbesondere ist es die Aufgabe der vorliegenden Erfindung ein Verfahren zur Herstellung kosmetischer Zubereitung bereit zu stellen, die eine neue kosmetische Produktform darstellt und dem Anwender ein neues Applikatianserlebnis bietet und das Anwendungsspektrum von Haut- und/oder Haarpflegeprodukten erweitert.

Gelöst wird dieses Bündel an Aufgaben durch ein Verfahren zur Herstellung kosmetischer und/oder dermatologischer Zubereitungen in Kapselform entsprechend den Hauptansprüchen. In den Unteransprüchen sind bevorzugte Ausführungsformen des Verfahrens offenbart.

Es war überraschend und für den Fachmann außerordentlich erstaunlich, dass die gestellten Aufgaben gelöst werden können durch ein Verfahren zur Herstellung kosmetischer und/oder dermatologischer Kapseln für kosmetische und/oder dermatologische Inhaltsstoffe umfassend eine Kapselhülle, die fest, halbfest und/oder formstabil ist und im wesentlichen aus Wachsen, Emulgatoren, natürlichen und/oder synthetischen Polymeren und/oder Mischungen daraus besteht.

Die Kapsel umfasst eine Füllung bestehend aus einem oder mehreren festen, halbfesten, pastösen und/oder flüssigen Inhaltsstoffen. Weiter bevorzugt ist, dass die Kapselhülle bis zu einer Temperatur von mindestens 35°C fest, halbfest und/oder formstabil Hülle ist.

Optional enthält die Kapselhülle zusätzlich Wasser und/oder Polyole.

Der Vorteil und gleichzeitig die erfindugsgemäße Eigenschaft der Kapsel ist, dass sie
- beim Verreiben und/oder Verteilen der Kapsel auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst
und so, insbesondere für den Anwender, nicht mehr, insbesondere als gesondert neben der Füllung vorliegender Bestandteil, wahrnehmbar ist.
D.h. die Kapsel zieht vorteilhafterweise bei der Applikation auf der Haut oder dem Haar vollständig ein ohne Rückstände zu hinterlassen.
Insbesondere gegenüber den bekannten kosmetischen Kapseln, deren Kapselhülle nach der Anwendung übrig bleibt, kann erfindungsgemäß die Kapselhülle vollständig auf der Haut verbleiben, so dass es z.B. möglich ist, Wirkstoffe in die Hülle einzuarbeiten. Damit kann die Kapselhülle einen aktiven, pflegenden Beitrag in der kosmetischen und dermatologischen Zubereitung leisten und dient nicht nur der Verpackung.

Viele Begriffe wie "Kugeln", "Kapseln" oder "kapselförmige Zubereitung" können grundsätzlich zur Beschreibung der erfindungsgemäßen Kapseln herangezogen werden, jedoch werden diesen Begriffen unter Umständen verschiedene Bedeutungen zugeordnet. Insbesondere die Bedeutung des Begriffes "Kapsel" ist hierin nicht auf die genau definierten Formen, Herstellungsverfahren, Inhaltsstoffe und Anwendungsmöglichkeiten der ebenfalls "Kapseln" genannten pharmazeutischen Präparate beschränkt, schließt diese aber mit ein. Generell ist erfindungsgemäß eine Kapsel ein beispielsweise ungefähr runder oder ellipsoider, von seiner Umgebung klar unterscheidbarer Gegenstand, der bei leichtem Druck und beispielsweise durch Anfassen bei der Entnahme aus einer Verpackung, seine Form nicht ändert. Erfindungsgemäß sind aber auch anderen Formen der Kapseln bzw. der Zubereitungen denkbar, sofern die beanspruchten Merkmale der Kapsel, der Kapselhülle und der Füllung eingehalten werden.

Die erfindungsgemäße Kapsel umfasst vorportionierte kosmetische Produkte, die aus einer festen oder halbfesten Hülle und vorteilhaft einer Füllung bestehen. Der Begriff fest bzw. halbfest definiert erfindungsgemäß den Zustand der Hülle, entsprechend der pharmazeutischen Technologie. Die feste bzw. halbfeste Hülle ist idealerweise auch als formstabil zu bezeichnen. Jedoch ist auch eine Kapsel mit einer, umgangssprachlich weichen, wabbeligen oder gallertartigen Hülle erfindungsgemäß. Die Hülle bricht, schmilzt und/oder löst sich beim Verreiben auf und löst sich ggf. beim Verteilen mit den Inhaltsstoffen der Füllung auf der Haut oder dem Haar auf. Damit ist gewährleistet, dass eine Zubereitung, beispielsweise als Kosmetikum ohne störende Rückstände auf der Haut oder dem Haar appliziert werden kann.

Es verbleiben gegenüber dem Stand der Technik bekannten kapselförmigen Präparaten keinerlei Hüllmaterialbestandteile ungenutzt auf der Haut zurück, die unansehnlich sind, unnötige Kosten und Umweltbelastungen verursachen und darüber hinaus nach der Produktapplikation vom Verbraucher entfernt werden müssen.

Die erfindungsgemäßen Kapseln weisen eine Größe, d.h. durchschnittlichen Durchmesser, von 5 bis 40 mm auf. Damit sind die Kapseln einzeln handhabbar und anwendbar.

Die erfindungsgemäßen kapselförmigen Zubereitungen sind als Dragees, Kapseln, Kugeln oder Hohlkugeln bei Lagerung und Entnahme formstabil und werden erst beim Verteilen flüssig bzw. lösen sich auf. Dies wird durch die spezielle Kombination des Hüll- bzw. in Kombination mit dem Kapselmaterial erreicht.

Die Kapselhülle muss unter Lagerungsbedingungen, denen kosmetische Produkte üblicherweise ausgesetzt sind, fest, halbfest und/oder formstabil sein, das heißt vorzugsweise die Form der erfindungsgemäßen Kapseln darf sich während der Lagerung nicht durch beispielsweise den Einfluss der Gravitation oder der Temperatur bis zu 35 °C verändern. Idealerweise kleben die Hüllen während der Lagerung auch dann nicht zusammen, wenn sich zwei Kapseln über längere Zeit berühren. Sollte dieses Erfordernis technologisch unpraktisch sein, lässt sich das Problem durch eine individuelle Umverpackung jeder einzelnen Kapsel, ähnlich einer Einzelverpackung (Bonbon-Papier) lösen.

Darüber hinaus muss das Hüllmaterial die Füllung während der Lagerung vor Austrocknung schützen. Dies trifft besonders zu, wenn die Füllung flüchtige Stoffe wie Wasser, kurzkettige Alkohole (z.B. Ethanol, Isopropanol), Parfum- und Riechstoffe oder niedrig siedende Öle (z.B. Cyclomethicon) enthält.

Das Hüllmaterial verbleibt nach der Anwendung vollständig auf der Haut. Erfindungsgemäß ist es von großer Bedeutung, dass alle Rohstoffe, aus denen die Hülle aufgebaut ist, sehr gut hautverträglich sind. Idealerweise leisten sie einen Beitrag zur Hautpflege, beispielsweise dadurch, dass sie die natürliche Hautbarriere stärken und die Haut so vor Austrocknung schützen.

Die Zusammensetzung der Hülle beeinflusst darüber hinaus maßgeblich das Hautgefühl des Verbrauchers bei Anwendung der erfindungsgemäßen Zubereitungen. Deshalb ist es vorteilhaft, die Hülle aus solchen Stoffen aufzubauen, die bei der Anwendung ein angenehmes Hautgefühl bewirken, sowie die sensorischen Eigenschaften der Hülle und der Füllung eng aufeinander abzustimmen.

Die hier beschriebenen technischen und sensorischen Anforderungen an die Hülle gehen deutlich über das übliche Anforderungsspektrum für bekannte kosmetische Zubereitungen hinaus. Deshalb ist es überraschend, dass sich erfindungsgemäße Hüllen mit Rohstoffen, wie sie dem Fachmanne bereits bekannt sind und in der Kosemtik bereits eingesetzt werden, herstellen lassen.

Es sind folgende Prinzipien bei der Kapsel und deren Hülle zu beachten.

Einerseits ist es möglich, bei Raumtemperatur flüssige Lipide oder bei Raumtemperatur flüssige Mischungen von Lipiden unterschiedlicher Schmelzbereiche durch Einbau von Wassertröpfchen (Herstellung einer W/O-Emulsion) derart zu verfestigen, dass die resultierende W/O-Emulsion auch über Raumtemperatur hinaus fest genug ist, um daraus eine erfindungsgemäße Hülle herzustellen. Andererseits können Lipide, deren Schmelzpunkt nahe an der Hauttemperatur von 32°C, also zwischen 30°C und 40°C liegt, zur Herstellung einer solchen erfindungsgemäßen Hülle verwendet werden. Drittens kann durch Verwendung geeigneter Wachse, polymerer Verdicker und/oder Gelbildner aus wässrigen und/oder Lipidsystemen eine thixotrope Zubereitung mit hoher Fließgrenze hergestellt werden, die den Anforderungen an die beschriebene Hülle genügt. Dem Fachmanne ist offensichtlich, dass die genannten Ansätze, Verwendung einer W/O- oder O/W-Emulsion, Verwendung von Lipiden mit vorteilhaften Schmelzpunkten, Verwendung thixotroper Systeme mit geeigneter Fließgrenze, beliebig miteinander kombiniert werden können, um die Eigenschaften der Hülle weiter zu optimieren.

Anhand dieser erfindungsgemäßen Vorgaben kann der Fachmann ohne selbst erfinderisch tätig zu werden eine erfindungsgemäße Kapsel herstellen. Die Kapselhülle ist somit durch die Eigenschaft charakterisiert, dass sie
- beim Verreiben und/oder Verteilen der Zubereitung auf der Haut und/oder dem Haar schmilzt und/oder
- aufgrund von Scherkräften völlig oder teilweise flüssig wird und/oder
- sich in der Füllung und/oder den Hautsebumlipiden bzw. durch Vermischung von Füllung und Hüllmaterial auflöst
und so insbesondere für den Anwender nicht mehr als gesondert neben der Füllung vorliegender Bestandteil der Zubereitung wahrnehmbar ist.

Mehrere erfindungsgemäße Kugeln, Hohlkugeln, Dragees oder Kapseln können gemeinsam in einer Packung aus Papier, Metall oder Plastik etc. oder einzeln oder zu mehreren durch weitere dünne Verpackungen ähnlich Bonbon-Papier oder in einer Blisterverpackung voneinander getrennt gelagert werden.

Vorteilhaft ist insbesondere die Kombination der kapselförmigen Zubereitungen mit einer Blisterverpackung, die während der Lagerung die einzelnen Dragees oder Kapseln voneinander trennt und so ein Zusammenfügen einzelner Kapseln durch beispielweise unsachgemäße Behandlung verhindert. Dies kann auch durch Umwickeln der einzelnen Dragees oder Kapseln mit dünnen Folien aus Papier, Metall oder Plastik erreicht werden. Außerdem können die Kapseln in Röhrchen z.B aus Polystyrol verpackt werden, oder in Folien eingesiegelt werden. Neben Folien aus Zellglas, Aluminium und Papier können auch Kunststofffolien verwendet werden. Im allgemeinen dient PE (Polymerisationsgrad von 3000-4000) als Material für derartige Packmittel. Weitere Möglichkeiten sind Durchdrückpackungen, bei denen auf eine kunststoffolie beispielsweise eine Aluminiumfolien aufgesiegelt ist, oder Schrumpfpackungen. Die Kapseln können z.B auch in Faltschachteln, Kartons, Dosen, oder Kunststofftüten eingebracht werden.

Die Entnahme einzelner erfindungsgemäßer Hohlkugeln, Dragees oder Kapseln kann durch einfaches Herausnehmen mit der Hand erfolgen. Es ist aber auch möglich, die Entnahme der erfindungsgemäßen Kapseln durch ein geeignetes Spendersystem zu erleichtern. Hierzu können z.B. durch Betätigen eines einfachen Mechanismus einzelne Hohlkugeln, Dragees oder Kapseln aus dem Spendersystem freigegeben werden. Beispiel hierfür sind die von der Firma PEZ International AG unter der Marke "PEZ" vertriebenen Spendersysteme für Bonbons und andere Süßwaren.

Vorteilhaft sind aber auch Spendersysteme bei denen auf einer runden Scheibe die Emulsionskapsein schneckenförmig in Vertiefungen eingelagert sind, und durch einen Dosiermechanismus einzeln zu entnehmen sind. Besonders vorteilhaft sind hierbei Ausführungen, die von ihrer äußeren Form her an bekannte Kosmetikprodukte, beispielsweise die bekannte NIVEA-Dose, erinnern, da dies die Gefahr einer Verwechselung mit Lebensmitteln reduziert.

Bei der Anwendung werden einzelne Dragees entnommen und auf der Haut verrieben. Das Produkt wird dabei durch Schmelzen, Scheren oder Lösen der festen oder halbfesten Hüll- oder Füllungsbestandteile niedriger viskos und gut verteilbar und löst sich auf bzw. in der Haut oder dem Haar auf. Dem Fachmanne ist offensichtlich, dass Hülle und/oder Füllung durchaus feste Bestandteile enthalten können, deren Lösen während der Applikation weder möglich noch erwünscht ist, nämlich Feststoffe, wie sie bereits in herkömmlichen Kosmetika eingesetzt werden, ohne dass der Verbraucher bemerkt, dass sie in fester Form vorliegen. Beispiele hierfür sind Füllstoffe, Lichtschutz- und Farbpigmente.

Der Anwender entnimmt eine oder mehrere der Kapseln und verreibt sie auf der Haut, wie er es ansonsten mit Hautcreme aus der Dose oder Tube gewohnt ist. Vorteilhaft ist hier jedoch dass er eine vorportionierte Menge ohne Überreste und Verpackung anwenden kann.

Der Vorteil der erfindungsgemäßen Kapseln ist die bequeme einfache Einmalanwendung für Zwischendurch. Ähnlich dem Schminken oder Einfetten der Lippen ist somit auch ein Eincremen oder die Hautpflege unterwegs möglich. Darüber hinaus kann der Verbraucher einzelne Kapseln auch anderen Verbrauchern anbieten. Dies ist bei herkömmlichen kosmetischen Produkten zwar auch möglich, aber das gemeinsame Benutzen beispielsweise einer Creme aus ein und demselben Tiegel gleicht psychologisch einer körperlichen Berührung. Deshalb existiert hier eine Hemmschwelle, die durch die vorliegende Erfindung beseitigt wird.

Es ist auch möglich, erfindungsgemäße Kapseln mit unterschiedlichen Eigenschaften (z.B. Parfum, Farbe, Hautgefühl, Lichtschutzfaktor, enthaltene Wirkstoffe, und Kombinationen dieser Eigenschaften) in einer Verpackung anzubieten, was bei herkömmlichen Hautpflegeprodukten nicht möglich ist.

Ein weiterer Vorteil ist, dass die Anwendung der erfindungsgemäßen Kapseln einigen Anwendern, vor allem Kindern, mehr Spaß macht als die Anwendung herkömmlicher kosmetischer Produkte. Dies kann es den Eltern erleichtern, ihre Kinder vor schädlichen Umwelteinflüssen wie beispielsweise UV-Strahlung zu schützen.

Dem Fachmann ist bekannt, dass ein großes Problem der Schutz kosmetischer Produkte vor Pilzen, Hefen und Bakterien ist, die während der Anwendung in das Produkt gelangen. Dies geschieht vor allem durch das Anfassen des Produktes durch den Verbraucher während der Entnahme. Es ist offensichtlich, dass die erfindungsgemäßen Kapseln hier einen weiteren Vorteil bieten, da der Verbraucher nur diejenigen Kapseln berührt, die er auch sofort anwendet. Die übrigen Kapseln bleiben so vor mikrobiellem Befall geschützt, weswegen im Vergleich zu herkömmlichen kosmetischen Produkten mit geringeren Gehalten an Konservierungsmitteln gearbeitet werden kann. Da Konservierungsmittel zu den weniger verträglichen kosmetischen Rohstoffen gehören, ist so eine verbesserte Verträglichkeit der Produkte erreichbar, was einen weiteren Vorteil der vorliegenden Erfindung darstellt.

Die kapselförmigen erfindungsgemäßen Zubereitungen können beliebige Formen haben, bevorzugt sind sie jedoch sphärisch mit einem Volumen von 0,1 bis 20 ml.

Die Hülle ist aufgebaut aus Wachsen, Emulgatoren, Polymeren oder Mischungen daraus, optional zusätzlich enthaltend Wasser und/oder Polyole. Die Hülle hat ausreichende Schlagstabilität, um mechanische Belastungen während Herstellung und Lagerung auszuhalten, und ist dünn genug, sich bei der Applikation schnell aufzulösen. Bevorzugt beträgt die Hülldicke 0,001 bis 3 mm, insbesondere Dicken im Bereich von 0,01-2 mm.

Auch der Einsatz von Wasser ist in Anteilen von 50% oder 60% problemlos möglich.

Die Hülle wird vorteilhaft aus Wachsen wie Ceresin, Ozokerit, Esterwachse, Glyceridwachse und/oder Fettalkoholen sowie festen Emulgatoren und deren Mischungen aufgebaut. Die Wachse können ihrer Herkunft nach Naturwachse, modifizierte Naturwachse, teilsynthetisch oder vollsynthetisch sein.

Alle Bestandteile werden so ausgewählt, dass sie die geforderte Form- und Temperaturstabilität gewährleisten, die Eintrocknung der Füllung durch Verdunsten verhindern und bei der Applikation schnell schmelzen, aufgrund von Scherkräften völlig oder teilweise flüssig werden oder sich im Füllmaterial auflösen.

Zur Optimierung der elastischen Eigenschaften können Polymere in die Hülle eingearbeitet werden. Geeignete Polymere sind Celluloseether, Polyvinylpyrrolidon, Polyacrylate oder Polymethacrylate, sowie Eudragit.

Das erfindungsgemäße Hüllmaterial setzt sich bevorzugt aus Wachsen zusammen, die aus der Gruppe der
- natürlichen Wachse, besonders bevorzugt Carnaubauwachs, Candelillawachs, Shellackwachs, Beerenwachs (Rhus Verniciflura), hydrierte Pflanzenöle, wie hydriertes Palm- oder Rapsöl, Bienenwachs, Wollwachs (Eucerit)
- Mono-, Di - und Triglyceride aus höheren gesättigten Fettsäuren mit 10 - 30 Kohlenstoffatomen oder Mischungen hieraus, besonders bevorzugt Glyceryltripalmitat (Dynasan 116) und/oder Glycerylstearat, Kahlwachs 6447 (Gemisch aus Fettsäureestern und Kohlenwasserstoffpolymer), Glyceryltribehenat (Syncrowax HRC)
- höheren gesättigten Fettalkohole, besonders bevorzugt solche mit 14 - 30 Kohlenstoffatomen, ganz besonders bevorzugt Stearylalkohol und/oder Behenylalkohol und/oder Cetylalkohol
- synthetischen Ester, bevorzugt C16-36 Alkylhydroxystearoylstearat, Stearylstearat, Cetearylbehenat, C20-40 Alkylstearat, besonders bevorzugt Cetylpalmitat, Methylpalmitat, Methylstearat, Myristylmyristat, Myristyllactat, Cetyllactat, Stearyllactat
- Polymerwachse, bevorzugt Polyethylen, Polypropylen, Polyvinylether, Polydecen besonders bevorzugt Polyvinylstearylether und hydriertes Polydecen,
- Copolymere, besonders bevorzugt solche aus Ethylen- und Vinylacetat sowie aus Polyvinylpyrrolidon und Hexadecen,
- Kohlenwasserstoffe/ Paraffinwachse, besonders bevorzugt Cera Microcristallina, Paraffinwachs, Ceresin, Ozokerit
- Silikonwachse
- chemisch modifizierten Wachse
- beliebigen Mischungen aus Wachsen der genannten Gruppen gewählt werden.

Vorteilhaft sind Hüllzusammensetzungen, die Rohstoffe und/oder Rohstoffkombinationen mit möglichst scharfen Schmelzpunkten oder engen Schmelzbereichen bei ca. 30°C enthalten. Dies können Einzelstoffe sein wie beispielsweise
- Ester von C12-C24-Fettsäuren mit kurzkettigen Alkoholen (C1-C6),
- Ester kurzkettiger organischer Säuren (C2-C6) mit mittel- oder langkettigen Fettalkoholen (C12-C24)
- Ester der Pyrrolidoncarbonsäure mit mittel- oder langkettigen Fettalkoholen (C12-C24)

Die genannten Säuren oder Alkohole können zusätzliche Gruppen umfassen wie z.B. Alkyl- oder Hydroxygruppen (z.B. Milchsäure).

Darüber hinaus können auch eutektische Gemische mit einer entsprechenden Eutektikumstemperatur verwendet werden, z.B. Mischungen von hydriertem Kokosfett mit Wachsestern (C12-C24 Fettsäure verestert mit C12-C24 Fettalkohol). Die Auswahl geeigneter eutektischer Gemische durch Untersuchung des Schmelzverhaltens von Mischungen (bspw. mittels Differential Scanning Calorimetry DSC) ist dem Fachmann bekannt.

Erfindungsgemäß besonders bevorzugte Wachse zur Herstellung der erfindungsgemäßen Hülle sind Cetylpalmitat, Cetylrizinoleat, Bienewachs, hydrierte Kokosglyceride, Methylpalmitat, Methylstearat, Methylstearat, Ethylstearat, Myristyllactat, Cetyllactat, Stearyllactat, Candelillawachs; Carnaubawachs, Paraffinwachs, Ceresin, Ozokerit, Myristylmyristat, Tripalmitin, Tribehenin, Glycerylpalmitostearat, hydriertes Rapsöl und C15-C40 Alkylstearylstearat.

Im Rahmen der vorliegenden Offenbarung wird als Oberbegriff für Fette, Öle, Wachse und dergleichen gelegentlich der Ausdruck "Lipide" verwendet, wie dem Fachmann durchaus geläufig ist. Auch werden die Begriffe "Ölphase" und "Lipidphase" synonym angewandt.

Wachse im Sinne der vorliegenden Erfindung weisen vorteilhaft einen Schmelzpunkt von 20 bis 90 °C, bevorzugt einen Schmelzpunkt von 25 bis 80 °C und besonders bevorzugt einen Schmelzpunkt von 30 bis 70 °C auf.

Um den Gesamtwachsgehalt der Kapseln zu reduzieren und so ein angenehmeres und ausgewogeneres Hautgefühl zu erreichen, kann es vorteilhaft sein, in die Wachshülle fein dispergierte Wassertröpfchen einzubauen, was beispielsweise im Bereich der Farbkosmetik und Lippenpflege Anwendung findet. Bei Raumtemperatur feste Lippenpflegestifte aus solchen W/O-Dispersionen werden beispielsweise in der Schrift DE 10148313 beschrieben. Diese dort beschriebene Technologie ist hiermit Gegenstand der vorliegenden Erfindung.

Zusätzlich können auch noch andere feste Stoffe in der Hülle enthalten sein, die zu allen Zeitpunkten (Herstellung, Lagerung, Anwendung) in fester Form vorliegen (z.B. anorganische Pigmente, Lipidverdicker auf Aerosil-, Hectorit-, Bentonit- oder allgemein Mineralbasis) oder über breite Schmelzbereiche oberhalb 30°C verfügen (natürliche Fette und Wachse wie Shea- oder Kokosbutter, Carnauba- oder Candelillawachs, gehärtete Fette wie hydriertes Kokosfett, Mikrokristalline Wachse, Ceresine etc.).

Die Füllung kann aus einem wasserhaltigen Medium, beispielsweise einer Emulsion (o/w, w/o, w/o/w), einem Gel, einer Mikroemulsion oder einer Hydrodispersion bestehen. Das Füllmaterial kann aus allen in der Kosmetik bekannten Stoffen und Zubereitungen bestehen, insbesondere O/W- oder W/O/W-Emulsionen in Form von Cremes oder wässrige Gele sind vorteilhaft.

Besonders vorteilhaft und damit erfindungsgemäß bevorzugt besteht die Hülle aus einer W/O-Emulsion und/oder Wachsen und die Füllung aus O/W-Emulsionen, wasserhaltigen Zusammensetzungen, Hydrogelen und/oder Hydrokolloiden. Diese Auswahl vermindert das gegebenenfalls mögliche Vermischen von Hüll- und Füllbestandteilen.

Hülle und Füllung können unabhängig voneinander die üblichen Hilfs- und Zusatzstoffe enthalten, die dem Fachmanne natürlich bekannt sind. Vorteilhaft ist dabei aber, dass die Zusatzstoffe unabhängig voneinander in der Hülle und/oder in der Füllung vorhanden sein können. Beispielsweise können somit Farbstoffe lediglich in der Hülle eingebracht werden ohne die Füllbestandteile zu verändern und dennoch ansprechende ästhetische Effekte erzielt werden. Die erfindungsgemäßen kosmetischen Zubereitungen können daher sowohl in der Hülle als auch in der Füllung kosmetische Hilfsstoffe enthalten, wie sie üblicherweise in Kosmetika verwendet werden, z.B. Konservieungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Geschmacksstoffe, Vergällungsmittel, Parfums, Verdickungsmittel, weichmachende Substanzen, anfeuchtende und/oder feuchthaltende Substanzen, Antioxidantien, UV-Filtersubstanzen, Sensorikadditive, Filmbildner, Tenside, Emulgatoren, Fette, Öle, Wachse, Wirkstoffe oder andere übliche Bestandteile einer kosmetischen Formulierung wie Alkohole, Polyole, stabilisierende Polymere, Schaumstabilisatoren, Elektrolyte, organische Lösungsmittel oder Silikonderivate.

Bevorzugte Emulgatoren sind O/W-Emuigatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethyleriglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ -H,
- der veretherten Fettsäureethoxylate der allgemeinen Formel

   R-COO-(-CH₂-CH₂-O-)ₙ -R',
- der veresterten Fettsäureethoxylate der allgemeinen Formel

   R-COO-(-CH₂-CH₂-O-)ₙ-C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel
   R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate.
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholettioxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylatelpropoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),

Polyethylenglycol(12)isostearylether (lsosteareth-12), Polyethylenglycol(13)isostearylether (lsosteareth-13), Polyethylenglycol(14)isostearylether (lsosteareth-14), Polyethylenglycol(15)isostearylether (lsosteareth-15), Polyethylenglycol(16)isostearylether (Isosteareth-16), Polyethylenglycol(17)isostearylether (lsosteareth-17), Polyethylenglycol-(18)isostearylether (lsosteareth-18), Polyethylenglycol(19)isostearylether (Isosteareth-19), Polyethylenglycol(20)isostearylether (lsosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Pofyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20),

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylerlglycol(24)stearat, Polyethylenglycol(25)stearat,

Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Besonders bevorzugte O/W-Emulgatoren sind Triceteareth-4-Phosphat, Polyglyceryl-3-Methylglucosedistearat, Polyethylenglycol-40-Stearat, Glycerylstearatcitrat, Ceteareth-20, Ceteareth-2, Cethyl-Dimethicone-Copolyol; Wollwachsalkohol, Methylglucosesesquistearat, PEG-PPG-Blockpolymere (Pluronics F68 / 127), Cetearylglucosid, Stearinsäure.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter oder unverzweigter Alkancarbonsäuren einer Kettenlänge von 8 bis 24, insbesondere 12 bis 20 Kohlenstoffatomen, Diglycerinester gesättigter und/oder ungesättigter, verzweigter oder unverzweigter Alkancarbonsäuren einer Kettenlänge von 8 bis 24, insbesondere 12 bis 20 Kohlenstoffatomen, Monoglycerinether gesättigter und/oder ungesättigter, verzweigter oder unverzweigter Alkohole einer Kettenlänge von 8 bis 24, insbesondere 12 bis 20 Kohlenstoffatomen, Diglycerinether gesättigter und/oder ungesättigter, verzweigter oder unverzweigter Alkohole einer Kettenlänge von 8 bis 24, insbesondere 12 bis 20 Kohlenstoffatomen, Polypropylenglykolester gesättigter und/oder ungesättigter, verzweigter oder unverzweigter Alkancarbonsäuren einer Kettenlänge von 8 bis 24, insbesondere 12 bis 20 Kohlenstoffatomen, Polyglycerylester gesättigter und/oder ungesättigter, verzweigter oder unverzweigter Alkancarbonsäuren einer Kettenlänge von 8 bis 24, insbesondere 12 bis 20 Kohlenstoffatomen, Sorbitanester gesättigter und/oder ungesättigter, verzweigter oder unverzweigter Alkancarbonsäuren einer Kettenlänge von 8 bis 24, insbesondere 12 bis 20 Kohlenstoffatomen Lanolinalkohol

Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 lsostearat, Polyglyceryl-2 Dipolyhydroxystearat, Cetyl PEG/PPG-10-1-Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, lsostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat.

Besonders bevorzugte W/O-Emulgatoren sind Poylethylenglycol-45/Dodecylglycolcopolymer, Polyglyceryl-3-Diisostearat, PEG-30 Dipolyhydroxystearate, Sorbitanisostearate, Sorbitan Stearate, Glyceryl Isostearate, Glyceryl Stearate und Sorbitan Oleate.

Es ist auch möglich, auf das Herabsetzen der Grenzflächenenergie durch Emulgatoren oder Tenside zu verzichten und statt dessen die Grenzfläche durch Anlagerung von in beiden Phasen unlöslichen Partikeln zu stabilisieren. Hierzu können natürliche oder künstliche Polymere (Polyethylen, Nylon, Stärke und ihre Derivate) oder anorganische Partikel (TiO₂ Al₂O₃, BaSO₄, BN, Silikate, Alumosilikate) verwendet werden.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, lsononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* erhältlich ist.

Es ist ferner bevorzugt, die Ölkomponenten aus der Gruppe lsoeikosan, Neopentylglykoldiheptanoat, Propylenglykoldicaprylat/dicaprat, Caprylic/Capric/Diglycerylsuccinat, Butylenglykol DicaprylatlDicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Sillkonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 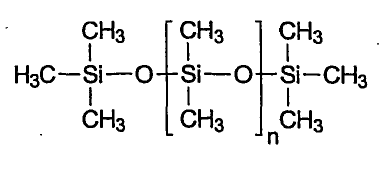 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die hier vortteilhaft beschriebenen Ölphasen können sowohl in der Hülle als auch in der Füllung vorhanden sein.

Es ist ebenfalls vorteilhaft, den Kapseln, der Hülle und/oder der Füllung im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Camosin und deren Derivate (z.B. Anserin), Carotinoide, Carotine (z.B. α-Carotin, β-Carotin, Lycopin) und deren Derivate, Retinoide wie zum Beispiel Retinol, Retinal und/oder Retinsäure und die jeweiligen Ester, α-Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoxirmin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Äpfelsäure), Huminsäure, Gallensäure, Gallenextrakte, Bilirubin, Biliverdin, EDTA, IDS, EGTA und deren Derivate, ungesättigte Fettsäuren und deren Derivate (z.B. γ-Linolensäure, Linolsäure, Ölsäure), Folsäure und deren Derivate, -2-Aminopropionsäurediessigsäure, Flavonoide, Polyphenole, Catechine, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Weiterhin können der erfindungsgemäßen Zubereitung UV-Filtersubstanzen zugesetzt werden. Es ist damit bevorzugt die erfindungsgemäßen Zubereitungen als Lichtschutzformulierungen anzuwenden.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure (2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂) Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlichbehandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aiuminiumstearat, pftanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Ebenfalls geeignete UV-A Filtersubstanzen sind Hydroxybenzophenone. Diese zeichnen sich durch die folgende Strukturformel aus: 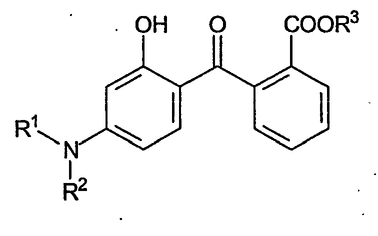 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 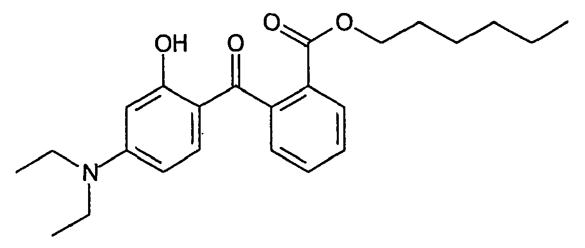 und unter dem Uvinul A Plus bei der Fa. BASF erhältlich ist.

Die Gesamtmenge an einem oder mehreren Hydroxybenzophenonen in den fertigen kosmetischen oder derffiatologischen Zubereitungen wird vorteilhaft aus dem Bereich 0,01 Gew.-% bis 20 Gew.-%, vorzugsweise von 0,1 bis 10 Gew.-% gewählt, jeweils bezogen auf das Gesamtgewicht der Zubereitungen. Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 oder unter Neo Heliopan Hydro bei erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX erhältlich;
- Sulfonsäure-Derivate des 3-Benzylidencamphers, wie z. B. 4-(2-Oxo-3-bomylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S erhältlich ist;
- Diethylhexylbutylamidotriazon (INCI: Diethylhexylbutamidotriazone), welches unter der Handelsbezeichnung UVASORB HEB erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb® M erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL erhältlich ist.

Die weiteren UV-Filtersubstanzen können öllöslich oder wasserlöslich sein.

Vorteilhafte öllösliche UV-B- und/oder Breitband-Filtersubstanzen im Sinne der vorliegenden Erfindung sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.
■ 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX erhältlich ist.

Vorteilhafte wasserlösliche Filtersubstanzen sind z. B.:
Sulfonsäure-Derivate des 3-Benzylidencamphers, wie z. B. 4-(2-Oxo-3-bomylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches unter der Bezeichnung Uvinul^{®} N 539 erhältlich ist.

Besonders vorteilhafte Zubereitungen im Sinne der vorliegenden Erfindung, die sich durch einen hohen bzw. sehr hohen UV-A- und/oder UV-B-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan], Phenylen-1,4-bis-(2-benzimidazyl)-3,3'-5,5'-tetrasulfonsäure und/oder ihre Salze, das 1,4-di(2-oxo-10-Sulfo-3-bomylidenmethyl)-Benzol und/oder dessen Salze und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschützmittel fürs Haar dienen.

Erfindungsgemäße Zubereitungen enthalten insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Vorteilhaft sind außerdem Taurate, z.B. Ammonium Acryloyldimethyltaurat / VP Copolymer.

Unter den Polyacrylaten befinden sich z.B. Carbopol Typen der Firma Goodrich (Carbopol 980, 981, 1382, 5984, 2984, ETD 2001, ETD 2020, ETD 2050, Ultrez-10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder - monoethylether, Ethylhexyloglycerin, Methylpropandiol und analoge Produkte, Polymere, Schaumstabilisatoren, Elektrolyte wie beispielsweise Natriumchlorid oder Magnesiumsulfat sowie insbesondere ein oder mehrere Verdickungsmittel, welches oder welche vorteilhaft gewählt werden können aus der Gruppe Siliziumdioxid, Alumosilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon, früher Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 erhältlich). Die Repellentien können sowohl einzeln als auch in Kombination eingesetzt werden.

Als Feuchthaltemittel und/oder hautbefeuchtende Stoffe werden Stoffe oder Stoffgemische bezeichnet, welche kosmetischen oder dermatologischen Zubereitungen die Eigenschaft verleihen, nach dem Auftragen bzw. Verteilen auf der Hautobertläche die Feuchtigkeitsabgabe der Hornschicht (auch transepidermal water loss (TEWL) genannt) zu reduzieren und/oder die Hydratation der Hornschicht positiv zu beeinflussen. Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Panthenol, Fucogel, Glycine Soja, Ethylhexyloxyglycerin, Pyrrolidoncarbonsäureund ihre Derivate sowie Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke-Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Neben der zuvor beschriebenen Anwendung der Kapseln als Sonnenschutz, Haut- und Lippenpflege, Selbstbräunung oder Insektenrepellent, wobei diesbezügliche Inhaltstoffe individuell gewählt werden können, ist die erfindungsgemäße Kapsel auch als Reinigungskapsel anwendbar.

In der Füllung finden sich dann Tenside oder waschaktive Substanzen, die beim Verreiben auf der Haut frei gesetzt werden. Die Einarbeitung als Füllbestandteil erfolgt dabei ohne jegliche Komplikationen.

Ein Problem bei der Hand- oder Gesichtsreinigung bestand darin, dass die Menge an Waschmitteln schwer zu dosieren war. Durch die tensidhaltigen Kapseln ist dem Anwender eine Möglichkeit gegeben, immer gleiche Mengen an Waschmittel einzusetzen. Dadurch das die Kapseln bei der Benutzung keine Rückstände hinterlassen, fällt keine zusätzliche Verpackung an.

Vorteilhaft ist insbesondere bei Bereitstellung der Kapsel mit Tensiden die Kombination der waschaktiven Substanzen in der Füllung mit dem Wachs in der Hülle. Somit hat man beim Auflösen der Kapseln beim Verreiben oder unter warmen Wasser eine Formulierung, die schäumt und durch den Wachsanteil in der Lage ist auch wasserfeste Rückstände, wie beispielsweise Make-up, zu entfernen.

Auf Reisen kann somit gezielt und zwischendurch das Gesicht und Haut gereinigt werden ohne das aufwendige Reinigungsmittel mitgenommen werden müssen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und -je nach Wunsch - für Schaumregulierung.

Bei den hydrophilen Anteilen eines Tensidmoleküls handelt es sich meist um polare funktionelle Gruppen, beispielweise -COO⁻, -OSO₃2-, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |

| | | |
|---|---|---|
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind

Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-Iauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

### Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

### Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Zur Anwendung werden die kapselförmigen Zubereitungen erfindungsgemäß in der für Kosmetika üblichen Weise auf die Haut und/oder die Haare aufgebracht und verrieben bzw. verteilt.

Der erfindungswesentliche Vorteil ist die erstmalige Bereitstellung einzelner handhabbarer kosmetischer Präparate in Kapselform, die dem Verbraucher eine vereinfachte Entnahme, verbesserte Hygiene beim Teilen des Produktes mit anderen sowie eine neue Applikationsform bieten.

Die erfindungsgemäßen kosmetischen und/oder dermatologischen Zubereitungen können wie übliche kosmetische und/oder dermatologische Zubereitungen zusammengesetzt sein und dem kosmetischen und/oder dermatologischen Lichtschutz, zur Änderung oder Beeinflussung bestimmter Hautzustände, ferner zur Behandlung, der Pflege und der Reinigung der Haut und/oder der Haare und als Schminkprodukt in der dekorativen Kosmetik dienen.

Entsprechend können kosmetische und/oder topische dermatologische Zusammensetzungen im Sinne der vorliegenden Erfindung, je nach ihrem Aufbau, beispielsweise verwendet werden als Hautpflegeprodukt, Hautschutzprodukt, Reinigungsprodukt, Sonnenschutzprodukt, Haarkur, Körperreinigungsprodukt, für die Tages- oder Nachtpflege, die Pflege bestimmter Hautareale wie Hände, Gesicht, Füße usw.

Auch ist die Verwendung der erfindungsgemäßen kapselförmigen kosmetischen Zubereitungen zur Prophylaxe und Behandlung der Symptome von Altershaut, zur Verhinderung und Verminderung der Entstehung und Ausbreitung von Fältchen und Falten sowie zur Behandlung und Pflege gealterter Haut erfindungsgemäß. So lässt sich vorteilhaft eine einzelne Kapsel, enthaltend Ubichinon, Ubichinol, Retinol und Derivate, Dehydroepiandrosteron (DHEA), Isoflavonoide (insbes. Genistein, Daidzein), Creatin, Phytoöstrogene, Östrogen, Östradiol und Derivate, Niacinamid, Polyphenole (AGR) oder eine andere, gegen Falten wirksame Substanz auf die Gesichtshaut auftragen und verteilen.

Weiterhin ist die Verwendung der erfindungsgemäßen kapselförmigen kosmetischen Zubereitungen zur Prophylaxe und Behandlung der Symptome trockener Haut bevorzugt. Geeignete Wirkstoffe für diesen Einsatzzweck sind: natürliche Öle (Sonnenblumen-, Nachtkerzensamen-, Jojoba-, Macadamiaöl, Rhizinusöl), Ceramide, insbes. Ceramid I, III und VI, Cholesterin, Phytosterole, Fettsäuren mit einer Kettenlänge von C16-26, Carnitin und seine Derivate, Harnstoff, Polyole wie Glycerin, Butylenglykol, Propylenglykol und Dipropylenglykol, Pseudoceramide; Elektrolyte wie Natriumchlorid und Taurin, Fettalkohole sowie Wachse.

Außerdem ist die Verwendung der erfindungsgemäßen kapselförmigen kosmetischen Zubereitungen zur Prophylaxe und Behandlung der Symptome der sensiblen bzw. entzündeten Haut vorteilhaft. Bevorzugte Wirkstoffe für diesen Einsatzzweck sind: Inhaltsstoffe der Mariendistel, insbesondere Silymarin, Hamamelisextrakt, Kamillenextrakt, Inhaltsstoffe der Süßholzpflanze (Glycerrhizinsäure, Licochalcone A & B), Allantoin, Acetylsalicylsäure, Diclofenac, Pentacyclische Triterpene (Sericoside, Ursolsäure) und Panthenol.

Außerdem ist die Verwendung der erfindungsgemäßen kapselförmigen kosmetischen Zubereitungen zur Prophylaxe und Behandlung der Symptome der fehlpigmentierten Haut vorteilhaft. Bevorzugte Wirkstoffe für diesen Einsatzzweck sind: Tyrosinaseinhibitoren, Hydrochinonderivate, Dioic Acid, Liponsäure und ihre Derivate sowie Kojisäure.

Es ist gegebenenfalls möglich und vorteilhaft, die erfindungsgemäßen Zusammensetzungen als Grundlage für pharmazeutische Formulierungen zu verwenden. Weiterhin ist die Verwendung der erfindungsgemäßen kapselförmigen kosmetischen Zubereitungen zur Prophylaxe und Behandlung der Symptome kranker Haut bevorzugt. Relevante aber nicht ausschließliche krankhafte Hautzustände sind Psoriasis, Akne, Neurodermitis und andere atopische Erkrankungen wie atopische Dermatitis, Hautkrebs, Herpes, Mykosen, Ichthyosis, Pityriasis, Seborrhöe, Pellagra, Kontaktekzeme und Allergien. Geeignete Wirkstoffe für solche Einsatzzwecke sind Antibiotika wie Fusidinsäure, Erythromycin, Sulfadiazin, Clindamycin, Tetracycline, Tyrothricin Aminoglycoside, Bacitracin, Chloramphenicol, Virustatika (z.B. Aciclovir, Idoxuridin, Penciclovir), Antimykotika (z.B. Nystatin, Amphotericin, Clotrimazol, Econazol, Ketoconazol,Naftifin, Terbinafin), Allethrin, Zytostatika (5-Fluorouracil), Antiphlogistica (Hydrocortison, Bethamethason; Prednisolon, Triamcinoionacetonid, Dexamethason, Diclofenac, Bufexamac), Immunosuppressiva (Cyclosporin A, Interferon-beta), Antipsoriatica (Dithranol, Psoralen, Tazaroten), Aknemittel (Retinsäure, Isotretinoin, Benzoylperoxid, Adapalen); Capsaicin, Azelainsäure, Keratolytika (Salicylsäure, Milchsäure), Antihistaminika (Azelastin, Levocabastin, Dinatrium-cromoglycin); Antipsoriatika (Dithranol, Calcitriol, Psoralen) und Vitamine (besonders die A-, B- und C-Vitamine).

Eine mögliche und erfindungsgemäß besonders vorteilhafte Anwendung ist es, Kapseln mit unterschiedlichen Verwendungszwecken in einer Packung anzubieten, beispielsweise solche zur Tages- und Nachtpflege, solche mit unterschiedlichen Farben, Düften, unterschiedlich starken Lichtschutzfaktoren oder unterschiedlichen Wirkstoffen. Es ist bei einer solchen Verwendung besonders vorteilhaft, die Kapseln unterschiedlicher Zusammensetzung durch unterschiedliche Form- und/oder Farbgebung für den Anwender unterscheidbar zu machen.

Nicht zuletzt ist die Verwendung der erfindungsgemäßen kosmetischen und/oder dermatologischen Zubereitungen zur Prophylaxe, Behandlung und Reinigung fettiger Haut sowie zur Prophylaxe und Behandlung von unreiner Haut sowie von Cellulite erfindungsgemäß.

Das erfindungsgemäßes Verfahren zur Herstellung der Kapseln bzw. deren Hülle verläuft nach dem Prinzip des frozen-cone bzw. cold-stamp Verfahrens wie sie beispielsweise in DE 19852262 oder DE 9321186 beschrieben sind.

Diese Verfahren aus der Lebensmitteltechnologie sind erfindungsgemäß auf die Herstellung kosmetischer Präparate angepasst worden.

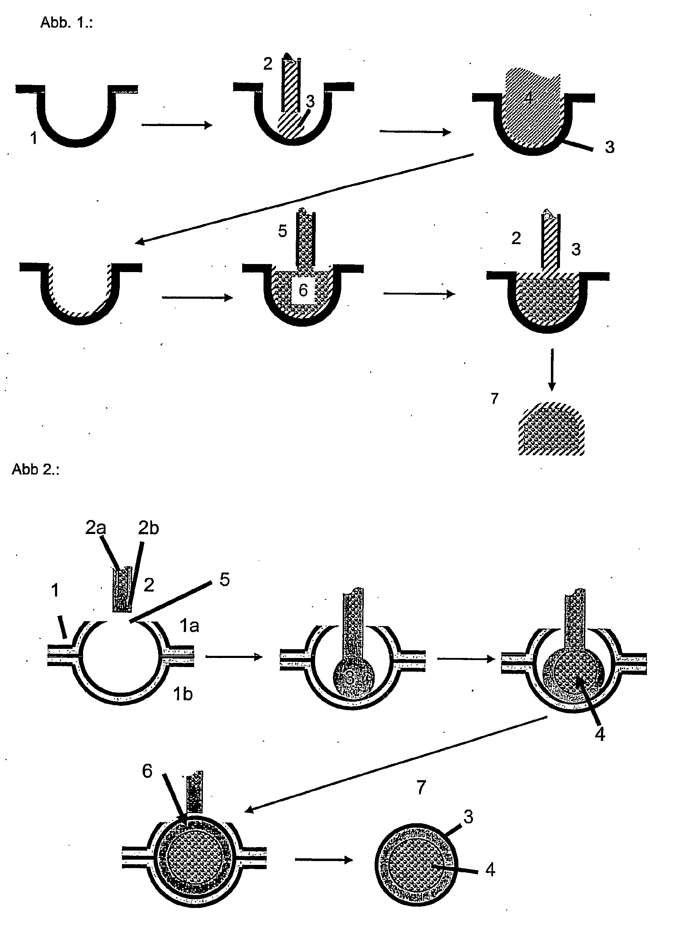

Das frozen-cone-Verfahren wird in der beigefügten Abbildung 1 verdeutlicht. In eine Form 1 wird durch eine Düse 2 zunächst eine definierte Menge Hüllmasse 3 gefüllt. Durch Einpressen eines gekühlten Formkörpers 4 (engl. "frozen cone" oder "cold stamp") wird die eingebrachte Hüllmasse geformt und gleichzeitig so gekühlt, dass sie bis zum Einfüllen der Füllmasse 6 durch eine weitere Düse 5 ihre Form nicht oder nur unwesentlich verändert. Nach dem Einbringen der Füllmasse wird durch die Düse 2a weitere Hüllmasse 3a aufgebracht. Die Düsen 2 und 2a und die Massen 3 und 3a können, müssen aber nicht identisch sein. Es ist vorteilhaft, die Massen 3 und 3a identisch zu wählen. Schließlich wird die fertige Emulsionskapsel 7 entformt.

Alternativ können nach dem Einbringen der Füllung zwei noch unverschlossene Halbkapseln aufeinander zur Deckung gebracht und ihre Hüllen miteinander verschmolzen werden.

Mit Hilfe dieses Verfahrens sind die Hülldicken mit hoher Reproduzierbarkeit im Bereich von 0,001 bis 3 mm variabel einstellbar. Gegenüber dem Stand der Technik sind damit erstmalig extrem dünnwandige Kapselpräparate herstellbar.

Ein weiterer Vorteil des Verfahrens ist, dass die nachträglich hinzuzufügende Füllung nicht erwärmt zu werden braucht. Dies ist besonders vorteilhaft, wenn die Füllung temperaturempfindliche Stoffe enthält, die bei erhöhten Temperaturen z.B. oxidativ (Vitamine A, B, C und E oder ihre Derivate wie Acetate, Phosphate oder Palmitate, Provitamin B5) oder hydrolytisch (Acetylcarnitin, Parabene) abgebaut werden. Natürlich kann durch die kalte Verarbeitung der Füllung auch Energie gespart werden, was die Herstellungskosten senkt und die Umwelt weniger belastet.

Um die erfindungsgemäßen Kapsel in verschiedensten Formen, Größe und Dicke der Hülle bereit stellen zu können, hat sich das ebenfalls aus dem Lebensmittelsektor bekannte one-shot-Verfahren bewährt. Auch dieses Verfahren wurde erfindungsgemäß für die Herstellung der erfindungsgemäßen kosmetischen Kapseln angepasst.

Dazu wird aus einer oberen und einer unteren Halbform (1a und b) eine Form 1 gebildet (vgl. Abbildung 2), wobei die obere Halbform 1a eine Öffnung 5 hat. Durch diese wird eine Doppeldüse 2, bei der ein innerer Kanal 2a konzentrisch in einem äußeren Kanal 2b angeordnet ist, in die Form 1 gefahren. Zunächst wird durch den äußeren Kanal etwas Hüllmasse 3 in die Form gefördert, sodass sich ein geschlossener Boden aus Hüllmasse bildet. Kurz darauf setzt auch die Förderung der Füllung 4 durch den inneren Kanal 2a ein. Während des Förderns der beiden Massen bewegt sich dabei die Düse 2 nach oben aus der Form 1 heraus. Abschließend wird die Förderung der Füllung wieder kurz ausgesetzt, damit auch ein geschlossener Deckel 6 entstehen kann. Durch anschließendes Trennen der beiden Halbformen 1a und b voneinander kann die fertige Emulsionskapsel 7 entformt werden.

Alternativ kann auch auf die obere Halbform 1a verzichtet werden, so dass die Öffnung 5 von der Öffnung bzw. deren offenen Seite der unteren Halbform 1b direkt gebildet wird. In die untere Halbform 1b werden die Massen 3 und 4 durch die Doppeldüse 2 direkt gefüllt, ähnlich dem Verfahren nach Abbildung 1.

So lassen sich zwar Kapseln aber nicht in Kugelform herstellen, sondern nur Körper mit einer planen Grundfläche (z.B. wie aus dem Lebensmittelsektor bekannte Schokoladen Praline Toffee-Fee oder Mozartkugeln). Aber auch diese Formen sind damit erfindungsgemäß.

Für das "one-shot"- äquivalente erfindungsgemäße Verfahren ist eine möglichst geringe Zügigkeit der Füllmasse notwendig. Unter Zügigkeit versteht man die Eigenschaft, bei der Entnahme eines Teils einer flüssigen oder halbfesten Zubereitung nicht sofort abzureißen, sondern Fäden zwischen dem entnommenen und dem unberührten Teil der Zubereitung zu ziehen. So ist beispielsweise geschmolzener Käse extrem zügig, reines Wasser hingegen ist nicht zügig, zieht also keine Fäden. Zieht nun die Füllmasse Fäden, so reißt sie auch nicht sauber ab, wenn ihre Förderung aufhört. Wegen der entstehenden Fäden kann sich kein vollständig geschlossener Deckel aus Hüllmaterial bilden, wodurch die Emulsionskapsel in ihren mechanischen (Formstabilität, Härte, Elastizität) und anderen Eigenschaften (Wasserverlust, Auslaufen der Füllmasse) nachteilig beeinflusst wird.

Kosmetische Rohstoffe, wie beispielsweise Polyole (besonders Glycerin, Propylenglykol, Butylenglykol, Polyethylenglykol, Pentandiole, Hexandiole, Octandiole) oder Hydrokolloide (besonders Polysaccharide wie Stärken und Stärkederivate, Mannane, Glucane, Xanthan Gum, Guar Gum, gummi arabicum, sowie Polymere oder Co-Polymere der Acrylsäure oder ihrer Ester), können die Zügigkeit der Füllung verstärken und sich so im Herstellverfahren negativ bemerkbar machen. Als Bestandteil der Füllung führt dies beim Befüllen der Hülle zum Fäden ziehen. Die Hülle kann dann nur noch mit Problemen verschlossen werden. Dieses Problem der Füllung kann überraschend durch eine Modifizierung der Hülle gelöst werden, indem der Hülle oberflächenaktive Substanzen, bevorzugt W/O-Emulgatoren zugesetzt werden. Bevorzugte W/O-Emulgatoren sind verzweigte oder unverzweigte, gesättigte oder ungesättigte Fettsäuren mit 12 bis 26 Kohlenstoffatomen, Polyglyceryl-3 Diisostearat, Polyglyceryl-4 Isostearat, Polyglyceryl-2 Dipolyhydroxystearat, Cetyl PEG/PPG-10-1- Dimethicone, PEG-30 Dipolyhydroxystearat, PEG-40 Sorbitanperisostearat, Cetyldimethiconecopolyol, PEG-7 Hydrogenated Castor Oil, PEG 45/Dodecylglycolcopolymer, PEG 22/Dodeceylglycolcopolymer, Pentaerythritylisostearat, Isostearyldiglycerylsuccinat, Sorbitanisostearat, Polyglyceryl-2 Sesquiisostearat, Glycerylisostearat, Sorbitanstearat, Glycerylstearat, PEG-25 Hydrogenated Castor Oil, PEG-40 Sorbitanperoleat, Sorbitanoleat, PEG-40 Sorbitanperisostearat, Polyglyceryl-3 Oleat, Polyglyceryl-2 Sesquioleat und Polyglyceryl-4 Isostearat. Darüber hinaus können auch Wachse mit Gehalten an oberflächenaktiven Substanzen verwendet werden, vor allem solche mit Gehalten an freien Fettsäuren und/oder Fettsäure-Mono- oder -diglyceriden (z.B. Bienenwachse). Sie erleichtern somit wesentlich das Vergießen von Füllungen, die Glycerin, Hydrokolloide und/oder die zuvor beschriebenen Stoffe enthalten. Der sich sonst bildende Faden wird durch den Zusatz der oberflächenaktiven Substanzen durch die Hülle durchtrennt. Der Zusatz gewährleistet somit ein problemloses Verschließen der Hülle.

Das Problem der Füllung (Fäden ziehen) wird erfindungsgemäß durch Hüllbestandteile gelöst.

Das frozen-cone oder cold-stamp-Verfahren zeichnet sich durch seine hervorragende Genauigkeit und Reproduzierbarkeit in der Dicke und Einheitlichkeit der Hülle aus. Es ist besonders gut geeignet, um sehr dünne Hüllen zu gießen (<1 mm).

Der Vorteil des one-shot-Verfahrens liegt in seiner außergewöhnlichen Flexibilität: Durch Einstellen einiger weniger maschineller Parameter lassen sich mit identischen Düsen, Maschinen und Formen Kapseln mit unterschiedlich dicken Hüllen herstellen. Für eine veränderte Form der Kapsel (bspw. oval statt rund) kann dieselbe Maschine verwendet werden, nur die Halbformen müssen ausgetauscht werden.

Die Formen, in die bei beiden Verfahren gegossen wird, können aus unterschiedlichen Materialien hergestellt werden, besonders bevorzugt thermoplastischen Kunststoffen wie Polyolefinen, Vinylpolymeren, Polyamiden, Polyestern, Polyacetalen und Polycarbonaten hergestellt werden. Vorteilhaft werden sie als Einmal-Formen hergestellt (Blister), die zur Verpackung der fertigen Kapsel verwendet werden können.

Beide zuvor erläuterten erfindungsgemäßen Verfahren ermöglichen eine industrielle, maschinengängige Herstellung der kapselförmigen kosmetischen Zubereitungen, so dass die Produktionskosten gesenkt werden und damit auch preiswerte kapselförmige Kosmetika angeboten werden können.

Die erfindungsgemäße Zubereitung weist zudem verbesserte sensorische Eigenschaften auf, die mit wachshaltigen Zubereitungen aus dem Stand der Technik nicht zu erwarten sind. Es wurde eine verbesserte Verteilbarkeit, Einziehungsvermögen, Konsistenz, Hautglättung und verminderte Klebrigkeit festgestellt. Bezüglich geeigneter Methoden zur Bestimmung dieser Parameter kann auf das Wissen des Fachmannes verwiesen werden.

Erstaunlich ist insbesondere, dass die erfindungsgemäße Kapsel ohne Füllung, als Hohlkugel, ebenso als eigenständiges Kosmetikum angewendet werden kann. Die für den kosmetischen oder dermatologischen Zweck enthaltenden Stoffe befinden sich dann erfindungsgemäß alle in der Kapselhülle.

Die Größenangaben wie beispielsweise der Durchmesser der Kapseln sind zu verstehen als Durchmesser in Richtung der Längsausdehnung der Kapselteilchen.

Nachfolgende Beispiele erläutern die erfindungsgemäßen kapselförmigen Zubereitungen. Die Prozentangaben beziehen sich soweit nichts anderes angegeben auf die Gesamtmasse der Zubereitungen.

### Beispiele:

A. Beispiele für die Zusammensetzung der Füllung (Angaben in Massenprozent bezogen auf das Gesamtgewicht der Füllung)

### Beispiele 1-5: O/W-Cremes

| **Beispiel** | **1** | **2** | **3** | **4** | **5** |
|---|---|---|---|---|---|
| Glycerylstearatcitrat | 2 | | | | |
| Glycerylstearat, selbstemulgierend | | 5 | 3 | 1 | 2 |
| PEG-40-Stearat | | | 1 | | 1 |
| Stearinsäure | | | | 4 | |
| Myristylmyristat | 1 | | | | 1 |
| Behenylalkohol | | | | 1 | |
| Stearylalkohol | 2 | 1 | | | |
| Cetearylalkohol | | | | | 2 |
| Cetylalkohol | 1 | | 3 | 3 | |
| Hydrierte Kokosfettglyceride (Hydrogenated Coco Glycerides) | 2 | | | | |
| Sheabutter | | 1 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 2 | 2 | 2 |
| Ethylhexylkokosfettsäureester | 3 | | | | 1 |
| Octyldodecanol | | | 1 | | |
| Jojobaöl | | | | 1 | |
| Mineralöl | | 1 | | | |
| Vaseline | 1 | | 1 | | 2 |

| **Beispiel** | **1** | **2** | **3** | **4** | **5** |
|---|---|---|---|---|---|
| Cyclomethicon | 3 | 2 | 4 | 3 | 5 |
| Dimethicon | | | 1 | 1 | |
| Dicaprylylether | 1 | 3 | 2 | | |
| Dicarprylylcarbonat | | | | 3 | |
| TiO₂ | 1 | | 1 | | 1 |
| Ethylhexylmethoxycinnamat | 5 | 3 | 5 | | 3 |
| Ethylhexyltriazon | | 1 | | | |
| Ethylhexylcyanodiphenylacrylat (Octocrylen) | | | | 5 | 3 |
| Butylmethoxydibenzoylmethan | 1 | | | | |
| Bis-Ethylhexyloxyphenol-methoxyphenyltriazine | | 1 | | | |
| Ethylhexylsalicylat | 1 | | | | |
| 2-Hydroxy-4-Methoxy-benzophenon (Oxybenzone) | | | | 2 3 2 | |
| Phenylbenzimidazol sulfonsäure | | | | 2 | |
| Ubichinon (Q10) | | | 0,03 | | |
| Tocopherylacetat | | | 1 | | 0,3 |
| Citronensäure, Natriumsalz | | 0.1 | | | |
| Creatin | 0,5 | | | | |
| Natrium-Ascorbylphosphat | | | | | 0,1 |

| **Beispiel** | **1** | **2** | **3** | **4** | **5** |
|---|---|---|---|---|---|
| Phenoxyethanol | 0,3 | | 0,3 | 0,2 | 0,2 |
| p-Hydroxybenzoesäurealkylester (Paraben) | 0,6 | 0,3 | 0,2 | 0,3 | 0,3 |
| Hexamidindiisethionat | | 0,04 | | | |
| Diazolidinylharnstoff | 0,25 | | 0,1 | 0,2 | 0,1 |
| 1,3-Dimethylol-5,5-dimethyl-hydantoin(DMDM Hydantoin) | | 0,2 | | | |
| Iodopropynylbutylcarbamat | | 0,1 | | | |
| Ethanol (vergällt) | | 2 | | | |
| Xanthan Gummi | | | 0,1 | | |
| Polyacrylsäure (Carbomer) | 0,05 | | | | 0,1 |
| Ammoniumpolyacryloyldimethyltaurate | 0,4 | | | 0,3 | |
| Ammoniumacryloyldimethyltauratel Vinylpyrrolidoncopolymere | | 0,5 | 0,3 | | |
| Aluminium Stärke Octenylsuccinate | | | | | 0,5 |
| Glycerin | 10 | 6 | 7 | 7 | 5 |
| Butylenglycol | | 1 | | 1 | |
| wasser- und/oder öllösliche Farbstoffe | 0,05 | | | | |
| Füllstoffe/ Additive (Distärkephosphat, SiO₂, BHT, Talkum, Aluminiumstearat) | 0,1 | 1 | 0,2 | 0,5 | 0,05 |
| Parfüm | q.s. | q.s. | q.s. | q.s. | q.s. |
| Wasser | ad 100 | ad 100 | ad 100 | ad 100 | ad 100 |

### Beispiel 6: Hydrodispersion/Gelcreme

| | |
|---|---|
| Cetylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Natriumascorbylphosphat | 0,05 |
| Iminodisuccinat | 0,2 |
| Ubichinon | 0,05 |
| Phenoxyethanol | 0,3 |
| p-Hydroxybenzoesäurealkyleste (Paraben) | 0,4 |
| Vernetztes Alkylacrylat (Alkylacrylate Crosspolymer) | 0,2 |
| Glycerin | 5 |
| Parfüm | q.s. |
| Wasser | ad 100 |

### B. Beispiele für die Zusammensetzung der Kapselhülle

### (Angaben in Massenprozent bezogen auf das Gesamtgewicht der Hülle)

### Beispiele 7-11: Wachshülle

| **Beispiel** | **7** | **8** | **9** | **10** | **11** |
|---|---|---|---|---|---|
| Myristylmyristat | 20 | | | | 5 |
| Ceresin | 5 | 5 | | | 4 |
| Cera Alba | 15 | | | | |
| Shea Butter | 10 | 15 | | | 7,5 |
| hydriertes Kokosfett | 20 | ad 100 | 10 | | 5 |
| Cera Microcristallina | 5 | | | | 5 |
| Glycerylstearatcitrat | | 20 | | | |
| Octyldodecanol | | | 15 | | 5 |
| Methylpalmitat | ad 100 | | | 25 | |
| Ozokerit | | 20 | | 10 | 5 |
| Hydriertes Polydecen | | | 15 | | |
| Polyethylene | | | 15 | 5 | |
| PEG-45/Dodecylglykol-Copolymer | | | 2 | | |
| Polyglyceryl-3-Diisostearat | | | 3 | | |
| Lanolin Alkohol | | | | 1 | 2 |
| Polyglyceryl-3-Diisostearat | | | | 1 | |
| PEG-40-Sorbitanperisostearat | | | | 3 | |
| Magnesiumstearat | | | | | 0,4 |
| Aluminiumstearat | | | | | 0,1 |
| Magnesiumsulfat | | | 1 | | |
| Natriumchlorid | | | | 1 | 1 |
| Konservierungsmittel | q. s. | q. s. | q. s. | q. s. | q. s. |
| Parfum | q. s. | q. s. | q. s. | q. s. | q. s. |
| Wasser | | | ad 100 | ad 100 | ad 100 |

### Beispiele 12-16: Wachshülle

| **Beispiel** | **12** | **13** | **14 1** | **5** | **16** |
|---|---|---|---|---|---|
| Myristylmyristat | | | 13 | | 5 |
| Ceresin | 5 | | | | 2 |
| Cera Alba | | | 10 | | |
| Shea Butter | | 10 | | 11 | |
| hydriertes Kokosfett | | 10 | | | |
| Cera Microcristallina | 5 | 3,5 | | 5 | |
| Octyldodecanol | | 10 | | | 5 9 |
| Methylpalmitat | 25 | | 5 | 7 | 20 |
| Ozokerit | | 10 | | 5 | |
| Hydriertes Polydecen | | | | 2 | |
| Polyethylene | 5,5 | | | | 4 |
| Polyglyceryl-3-Diisostearat | | | | 3 | |
| Lanolin Alkohol | 0,6 | | 0,5 | 0,5 | |
| Magnesiumstearat | 0,4 | | | 0,5 | |
| Aluminiumstearat | 0,01 | | | | |
| Polyglyceryl-3 Diisostearat | 2,5 | | | | |
| PEG-30-Dipolyhydroxystearat | | 1,5 | | | |
| PEG-7 hydrierstes Ricinusöl | | | 0,5 | | |
| Polyglyceryl-2 Dipolyhydroxystearat | | | | 1 | |
| Bornitrid | | | | | 5 |
| Magnesiumsulfat | 1 | | 1 | | 1 |
| Natriumchlorid | | | | 1 | |
| Konservierungsmittel | q. s. | q. s. | q. s. | q. s. | q. s. |
| Parfum | q. s. | q. s. | q. s. | q. s. | q. s. |
| Wasser | ad 100 | ad 100 | ad 100 | ad 100 | ad 100 |

### Beispiele 17-21: Wachshülle

| **Beispiel** | **17** | **18** | **19** | **20** | **21** |
|---|---|---|---|---|---|
| Myristylmyristat | | | 20 | | 5 |
| Cera Alba | 20 | | | | |
| Shea Butter | | 20 | | | 7,5 |
| hydriertes Kokosfett | 20 | ad 100 | 10 | | 9 |
| Cera Microcristallina | 5 | | | | 5 |
| Glycerylstearatcitrat | | 20 | | | |
| Methylpalmitat | ad 100 | | | 10 | |
| Cetylrizinoleat | 10 | | | 15 | 5 |
| Ozokerit | | 20 | | 10 | 5 |
| Hydriertes Polydecen | | | 15 | | |
| Polyethylene | 5 | | 15 | 5 | |
| Glycerylstearat | | | 2 | | |
| Polyglyceryl-3-Diisostearat | | | 3 | | |
| Lanolin Alkohol | | | | | 1 2 |
| Polyglyceryl-3-Diisostearat | | | | 1 | |
| Sorbitanstearat | | | 1 | 3 | |
| Magnesiumstearat | | | | | 0,4 |
| Aluminiumstearat | | | | | 0,1 |
| Magnesiumsulfat | . | | 1 | | |
| Natriumchlorid | | | | 1 | 1 |
| TiO2 | | 2 | | 2 | |
| Ethylhexylmethoxycinnamat | 5 | | 5 | | |
| PVP | | | | 1 | |
| PVP-Hexadecen-Copolymer | | 1 | | | |
| Methylhydroxypropylcellulose | | | 2 | | |
| Ethylcellulose | 2 | | | | 1 |
| Butylmethoxydibenzoylmethan | 1 | | 2 | | |
| Dimethiconcopolyol | | | | | |
| Glycerin | | | 10 | 5 | 15 |
| Konservierungsmittel | q. s. | q. s. | q. s. | q. s. | q. s. |
| Parfum | q. s. | q. s. | q. s. | q. s. | q. s. |
| Wasser | | | ad 100 | ad 100 | ad 100 |

### Beispiele 22-25: Wachshülle

| **Beispiel** | **22** | **23** | **24** | **25** |
|---|---|---|---|---|
| Myristylmyristat | | | 13 | |
| Cera Alba | | | 10 | |
| Shea Butter | | 10 | | 11 |
| hydriertes Kokosfett | | 10 | | |
| Cera Microcristallina | 5 | 3,5 | | 5 |
| Octyldodecanol | | 10 | | 5 |
| Methylpalmitat | 30 | | | 5 7 |
| Ozokerit | | 10 | | 5 |
| Hydriertes Polydecen | | | | 2 |
| Polyethylene | 5,5 | | | |
| Polyglyceryl-3-Diisostearat | 2,5 | 3 | | 3 |
| Lanolin Alkohol | 0,6 | | 0,5 | 0,5 |
| Magnesiumstearat | 0,4 | | | |
| Aluminiumstearat | 0,01 | | | |
| Bentonit | | | | 1 |
| Cetearylalkohol | | 1,5 | | |
| PEG-7 hydrierstes Ricinusöl | | | 0,5 | |
| Polyglyceryl-2 Dipolyhydroxystearat | | | | 1 |
| TiO2 | 1 | | | |
| Ethylhexylmethoxycinnamat | 3 | | 2 | |
| PVP | | | | |
| PVP-Hexadecen-Copolymer | 1 | | | |
| Methylhydroxypropylcellulose | | 1 | | |
| Ethylcellulose | 1 | 1 | 1 | |
| Butylmethoxydibenzoylmethan | 2 | | 1 | |
| Dimethiconcopolyol | | | | 1 |
| Glycerin | | 5 3 | | 1 |
| Magnesiumsulfat | 1 | | 1 | |
| Natriumchlorid | | | | 1 |
| Konservierungsmittel | q. s. | q. s. | q. s. | q. s. |
| Parfum | q. s. | q. s. | q. s. | q. s. |
| Wasser | ad 100 | ad 100 | ad 100 | ad 100 |

Beispiele für besonders bevorzugte Rezepturbeispiele für W/O-Hüllmassen (Angaben in Gew.% bezogen auf die jeweilige Masse)

| **Beispiel** | **26** |
|---|---|
| Methylpalmitat | 15,0 |
| Mikrokristallines Wachs | 10,0 |
| Polyethylen | 5,0 |
| PEG-40 Sorbitan Perisostearate | 2,0 |
| Eucerit | 0,5 |
| Polyglyceryl-3 Diisostearate | 1,0 |
| Glycerin | 5,0 |
| MgSO₄ | 0,2 |
| lodopropynyl Butylcarbamate | q.s. |
| Parabene | q.s. |
| Parfum | q.s. |
| Wasser | ad 100 |

| **Beispiel** | **27** |
|---|---|
| Methylstearat | 20,0 |
| Mikrokristallines Wachs | 8,0 |
| Carnaubawachs | 7,0 |
| C12-C15 Alkylbenzoat | 3,0 |
| Polyglyceryl-2 Dipolyhydroxystearate | 2,0 |
| Polyglyceryl-3 Diisostearate | 1,0 |
| Glycerin | 6,0 |
| MgSO₄ | 0,3 |
| hydrophobisierte Silica | q.s. |
| Phenoxyethanol | q.s. |
| Parabene | q.s. |
| Parfum | q.s. |
| Wasser | ad 100 |

| **Beispiel** | **28** |
|---|---|
| Ethylpalmitat | 18,0 |
| C18-38 Alkyl Hydroxystearoyl Stearate | 5,0 |
| Hydriertes Kokosfett | 10,0 |
| Macadamiaöl | 5,0 |
| Polyglyceryl-2 Dipolyhydroxystearate | 2,0 |
| Polyglyceryl-3 Diisostearate | 1,0 |
| Glycerin | 4,0 |
| MgSO₄ | 0,3 |
| Sorbinsäure | q.s. |
| Parabene | q.s. |
| Parfum | q.s. |
| Wasser | ad 100 |

| **Beispiel** | **29** |
|---|---|
| Ethylstearat | 15,0 |
| Ceresin | 10,0 |
| Cetylpalmitat | 5,0 |
| Paraffinöl | 5,0 |
| Polyglyceryl-2 Dipolyhydroxystearate | 2,0 |
| Polyglyceryl-3 Diisostearate | 1,0 |
| Glycerin | 8,0 |
| MgSO₄ | 0,3 |
| Milchsäure | q.s. |
| Aluminum Starch Octenylsuccinate | q.s. |
| Parabene | q.s. |
| Parfum | q.s. |
| Wasser | ad 100 |

| **Beispiel** | **30** |
|---|---|
| Myristyllactat | 25,0 |
| Ceresin | 10,0 |
| Myristylmyristat | 5,0 |
| Polyethylen | 3,0 |
| Shea Butter | 1,0 |
| Butyl Methoxydibenzoylmethane | 1,0 |
| PEG-30 Dipolyhydroxystearate | 3,0 |
| Glycerin | 10,0 |
| MgSO₄ | 0,3 |
| Nylon | q.s. |
| Iodopropynyl Butylcarbamate | q.s. |
| Parabene | q.s. |
| Parfum | q.s. |
| Na-EDTA | q.s. |
| Wasser | ad 100 |

| **Beispiel** | **31** |
|---|---|
| Cetyllactat | 18,0 |
| Ceresin | 2,0 |
| Mikrokristallines Wachs | 4,0 |
| Polyethylen | 6,0 |
| Ethylhexyl Methoxycinnamate | 1,0 |
| PEG-30 Dipolyhydroxystearate | 3,0 |
| Glycerin | 6,0 |
| MgSO₄ | 0,3 |
| hydrophobisierte Silica | q.s. |
| DMDM Hydantoin | q.s. |
| Parabene | q.s. |
| Parfum | q.s. |
| Wasser | ad 100 |

| **Beispiel** | **32** |
|---|---|
| Lauryl PCA | 20,0 |
| Ceresin | 6,0 |
| Cetylpalmitat | 3,0 |
| Candelillawachs | 1,0 |
| Dimethicone | 2,0 |
| PEG-30 Dipolyhydroxystearate | 4,0 |
| Glycerin | 6,0 |
| MgSO₄ | 0,3 |
| hydrophobisierte Silica | q.s. |
| Phenoxyethanol | q.s. |
| Parabene | q.s. |
| Parfum | q.s. |
| Wasser | ad 100 |

| **Beispiel** | **33** |
|---|---|
| Myristylmyristat | 11,0 |
| hydriertes Koksfett | 9,0 |
| Mikrokristallines Wachs | 5,0 |
| Polyethylen | 3,0 |
| Dicaprylylcarbonat | 5,0 |
| Polyglyceryl-2 Dipolyhydroxystearate | 1,0 |
| Polyglyceryl-3 Diisostearate | 2,0 |
| Glycerin | 4,0 |
| MgSO₄ | 0,3 |
| Mica | q.s. |
| Phenoxyethanol | q.s. |
| Parabene | q.s. |
| Parfum | q.s. |
| Wasser | ad 100 |

Darüber hinaus können die Hülle oder Füllung unabhängig voneinander Hilfsstoffe wie UV-Filter, Wirkstoffe, Sensorikadditive, Verdicker, Gelbildner, Farbstoffe, Farb-, Effekt-, oder UV-Pigmente Konservierungsmittel Antioxidantien, Komplexbildner Geschmacksstoffe, Vergällungsmittel oder Parfum enthalten.

Wie beschrieben können die verschiedenen Füllungen von einer der beispielhaft aufgeführten Hülle umgeben werden. Die Auswahl der beispielhaft dargestellten Füllungen und Hüllen ist vom jeweiligen Anwendungszweck abhängig.

## Patentansprüche

1. Verfahren zur Herstellung kosmetischer und/oder dermatologischer Kapseln umfassend eine Kapselhülle, die fest, halbfest und/oder formstabil ist und im Wesentlichen aus Wachsen, Emulgatoren, natürlichen und/oder synthetischen Polymeren und/oder Mischungen daraus besteht und
einer Füllung, bestehend aus einem oder mehreren festen, halbfesten, pastösen und/oder flüssigen kosmetischen und/oder dermatologischen Inhaltsstoffen, das durch folgende Schritte **gekennzeichnet** ist,
- Füllen der Hüllmasse (3) durch eine Düse (2) in eine Form (1),
- Einpressen eines gekühlten Formkörpers (4) in die Hüllmasse (3),
- Formen und Kühlung der eingebrachte Hüllmasse (3),
- Einfüllen der Füllmasse (6) durch eine weitere Düse (5),
- Aufbringen weiterer Hüllmasse (3a) durch Düse (2a), wobei die Düsen (2) und (2a) und die Massen (3) und (3a) identisch sein können,
- Verschließen der Hülle (3) und entformen der fertigen Kapsel (7).

2. Verfahren zur Herstellung kosmetischer und/oder dermatologischer Kapseln umfassend eine Kapselhülle, die fest, halbfest und/oder formstabil ist und im Wesentlichen aus Wachsen, Emulgatoren, natürlichen und/oder synthetischen Polymeren und/oder Mischungen daraus besteht und
einer Füllung, bestehend aus einem oder mehreren festen, halbfesten, pastösen und/oder flüssigen kosmetischen und/oder dermatologischen Inhaltsstoffen, das durch folgende Schritte **gekennzeichnet** ist,
- Bildung einer Form (1) aus einer oberen (1 a) und einer unteren Halbform (1 b), wobei die obere Halbform eine Öffnung (5) hat,
- Einführen einer Doppeldüse (2), bei der ein innerer Kanal (2a) konzentrisch in einem äußeren Kanal (2b) angeordnet ist, durch die Öffnung (5) in die Form (1),
- Einfördern von etwas Hüllmasse (3) durch den äußeren Kanal (2b) in die Form (1), so dass sich ein geschlossener Boden aus Hüllmasse bildet,
- Förderung der Füllung (4) durch den inneren Kanal (2a) in die Form (1), wobei
- sich während des Fördems der beiden Massen (3, 4) die Düse (2) nach oben aus der Form (1) herausbewegt,
- Aussetzen der Förderung der Füllung (4), damit eine geschlossene Hülle entstehen kann (6),
- abschließendes Trennen der beiden Halbformen (1a und b) voneinander und entformen der fertigen Kapsel (7).

3. Verfahren nach Anspruch 2, **dadurch gekennzeichnet, dass** die Form (1) nur durch eine untere Halbform (1 b) mit einer offenen Oberfläche oder Öffnung (5) gebildet ist.

4. Verfahren nach Anspruch 1 bis 3, **dadurch gekennzeichnet, dass** die Kapselhülle bis zu einer Temperatur von mindestens 35°C fest, halbfest und/oder formstabil ist.

5. Verfahren nach einem der vorstehenden Ansprüche, **dadurch gekennzeichnet, dass** die Kapselhülle aus Wachsen, gewählt aus mikrokristallinen Wachsen, Paraffinwachsen, Esterwachsen, Glyceridwachsen, und/oder Fettalkoholen, festen Emulgatoren und deren Mischungen besteht.

6. Verfahren nach einem der Ansprüche 1 bis 5, **dadurch gekennzeichnet, dass** die Kapselhülle zusätzlich Wasser, bevorzugt zu einem Anteil von 50 bis 60 Gew.%, und/oder Polyole enthält.

7. Verfahren nach einem der vorstehenden Ansprüche, **dadurch gekennzeichnet, dass** die Kapselhülle bei Raumtemperatur flüssige Lipide oder flüssige Mischungen davon enthält und durch Einbau von Wassertröpfchen verfestigt ist.

8. Verfahren nach einem der vorstehenden Ansprüche, **dadurch gekennzeichnet, dass** die Kapselhülle aus einer formstabilen Lipid-/Emulgatormischung besteht, die dispergiertes Wasser mit einer Tröpfchengröße unter 50 Mikrometer enthält.

9. Verfahren nach einem der vorstehenden Ansprüche, **dadurch gekennzeichnet, dass** die Kapsel einen durchschnittlichen Durchmesser von 3 bis 40 mm aufweisen.

10. Verfahren nach einem der vorstehenden Ansprüche, **dadurch gekennzeichnet, dass** die Kapsel überwiegend sphärische, runde oder elipsoide Formen mit einem Volumen von 0,1 bis 20 ml aufweisen.

11. Verfahren nach einem der vorstehenden Ansprüche, **dadurch gekennzeichnet, dass** die Kapselhülle eine Dicke von 0,001 bis 3 mm, bevorzugt 0,01 bis 2 mm, besitzt.

12. Verfahren nach einem der vorstehenden Ansprüche, **dadurch gekennzeichnet, dass** der oder die Inhaltsstoffe der Füllung gewählt werden aus der Gruppe wasserhaltiger Zubereitungen, O/W-, W/O-, W/O/W-Emulsionen, Gele, Hydrodispersionen, Tenside in fester oder flüssiger Form, Mikroemulsionen und/oder Mischungen daraus.

13. Verfahren nach einem der vorstehenden Ansprüche, **dadurch gekennzeichnet, dass** die Füllung waschaktive Substanzen und/oder Tenside in fester oder flüssiger Form enthält und die Hüllbestandteile Wachse umfassen.

## Claims

1. Method of producing cosmetic and/or dermatological capsules comprising an envelope which is solid, semisolid and/or dimensionally stable and consists essentially of waxes, emulsifiers, natural and/or synthetic polymers and/or mixtures thereof and a filling, consisting of one or more solid, semisolid, pasty and/or liquid cosmetic and/or dermatological ingredients which is **characterized by** the following steps,
- filling of the enveloping mass (3) through a nozzle (2) into a mold (1),
- pressing a chilled shaped body (4) into the enveloping mass (3),
- shaping and cooling the introduced enveloping mass (3),
- introducing the filling mass (6) through a further nozzle (5),
- applying further enveloping mass (3a) through nozzle (2a), where nozzles (2) and (2a) and masses (3) and (3a) may be identical,
- sealing the envelope (3) and removing the finished capsule (7).

2. Method of producing cosmetic and/or dermatological capsules comprising an envelope which is solid, semisolid and/or dimensionally stable and consists essentially of waxes, emulsifiers, natural and/or synthetic polymers and/or mixtures thereof and a filling, consisting of one or more solid, semisolid, pasty and/or liquid cosmetic and/or dermatological ingredients, which is **characterized by** the following steps,
- formation of a mold (1) from an upper half-mold (1a) and a lower half-mold (1b), where the upper half-mold has an opening (5),
- inserting a twin nozzle (2) in which an inner channel (2a) is arranged concentrically in an outer channel (2b), through the opening (5) into the mold (1),
- conveying some enveloping mass (3) through the outer channel (2b) into the mold (1) so that a continuous base of enveloping mass is formed,
- conveying the filling (4) through the inner channel (2a) into the mold (1), where
- during the conveying of the two masses (3, 4) the nozzle (2) is moved upward out of the mold (1),
- stopping the conveyance of the filling (4) so that a sealed envelope can form (6),
- final separation of the two half-molds (1a and b) from one another and release of the finished capsule (7).

3. Method according to claim 2, **characterized in that** the mold (1) is formed only by a lower half-mold (1b) with an open surface or opening (5).

4. Method according to claims 1 to 3, **characterized in that** the envelope is solid, semisolid and/or dimensionally stable up to a temperature of at least 35°C.

5. Method according to one of the preceding claims, **characterized in that** the envelope consists of waxes chosen from microcrystalline waxes, paraffin waxes, ester waxes, glyceride waxes, and/or fatty alcohols, solid emulsifiers and mixtures thereof.

6. Method according to one of claims 1 to 5, **characterized in that** the envelope additionally comprises water, preferably in an amount of from 50 to 60% by weight, and/or polyols.

7. Method according to one of the preceding claims, **characterized in that** the envelope comprises lipids which are liquid at room temperature or liquid mixtures thereof and is solidified by incorporating water droplets.

8. Method according to one of the preceding claims, **characterized in that** the envelope consists of a dimensionally stable lipid/emulsifier mixture which comprises dispersed water with a droplet size below 50 micrometers.

9. Method according to one of the preceding claims, **characterized in that** the capsules have an average diameter of from 3 to 40 mm.

10. Method according to one of the preceding claims, **characterized in that** the capsules have predominantly spherical, round or ellipsoidal shapes with a volume of from 0.1 to 20 ml.

11. Method according to one of the preceding claims, **characterized in that** the envelope has a thickness of from 0.001 to 3 mm, preferably 0.01 to 2 mm.

12. Method according to one of the preceding claims, **characterized in that** the ingredient or the ingredients of the filling are chosen from the group of aqueous preparations, O/W, W/O, W/O/W emulsions, gels, hydrodispersions, surfactants in solid or liquid form, microemulsions and/or mixtures thereof.

13. Method according to one of the preceding claims, **characterized in that** the filling comprises washing-active substances and/or surfactants in solid or liquid form and the envelope constituents include waxes.

## Revendications

1. Procédé pour la fabrication de capsules cosmétiques et/ou dermatologiques comprenant une enveloppe de capsule qui est solide, semi-solide et/ou à stabilité dimensionnelle et est essentiellement constituée de cires, d'émulsifiants, de polymères naturels et/ou synthétiques et/ou de mélanges de ceux-ci et
d'un remplissage constitué d'un ou plusieurs composants cosmétiques et/ou dermatologiques solides, semi-solides, pâteux et/ou liquides, qui est **caractérisé par** les étapes suivantes ;
- introduction de la matière d'enveloppe (3) par une buse (2) dans un moule (1),
- enfoncement d'un corps de formage (4) refroidi dans la matière d'enveloppe (3),
- mise en forme et refroidissement de la matière d'enveloppe (3) introduite,
- injection de la matière de remplissage (6) par une autre buse (5),
- application d'une autre matière d'enveloppe (3a) par la buse (2a), les buses (2) et (2a) pouvant être identiques et les matières (3) et (3a) pouvant être identiques,
- fermeture de l'enveloppe (3) et démoulage de la capsule finale (7).

2. Procédé pour la fabrication de capsules cosmétiques et/ou dermatologiques comprenant une enveloppe de capsule qui est solide, semi-solide et/ou à stabilité dimensionnelle et est essentiellement constituée de cires, d'émulsifiants, de polymères naturels et/ou synthétiques et/ou de mélanges de ceux-ci et
d'un remplissage constitué d'un ou plusieurs composants cosmétiques et/ou dermatologiques solides, semi-solides, pâteux et/ou liquides, qui est **caractérisé par** les étapes suivantes :
- formation d'un moule (1) à partir d'un demi-moule supérieur (1a) et d'un demi-moule inférieur (1b), le demi-moule supérieur comportant une ouverture (5)
- introduction d'une double buse (2), dans laquelle un canal interne (2a) est disposé coaxialement dans un canal externe (2b), à travers l'ouverture (5) dans le moule (1),
- descente d'un peu de matière d'enveloppe (3) à travers le canal externe (2b) dans le moule (1), de sorte qu'il se forme un fond continu de matière d'enveloppe,
- introduction de la matière de remplissage (4), par le canal interne (2a), dans le moule (1),
- la buse (2) étant relevée hors du moule (1) vers le haut au cours du transport des deux matières (3, 4),
- arrêt du transport de la matière de remplissage (4) afin qu'une enveloppe fermée puisse en résulter (6),
- séparation finale des deux demi-moules (1a et b) l'un de l'autre et démoulage de la capsule (7) finale.

3. Procédé selon la revendication 2, **caractérisé en ce que** le moule (1) n'est constitué que par un demi-moule (1b) présentant une ouverture ou surface ouverte (5).

4. Procédé selon l'une quelconque des revendications 1 à 3, **caractérisé en ce que** l'enveloppe de capsule est solide, semi-solide et/ou à stabilité dimensionnelle jusqu'à une température d'au moins 35 °C.

5. Procédé selon l'une quelconque des revendications précédentes, **caractérisé en ce que** l'enveloppe de capsule est constituée de cires, choisies parmi des cires microcristallines, des cires de paraffines, des cires d'esters, des cires de glycérides, et/ou d'alcools gras, d'émulsifiants solides et de mélanges de ceux-ci.

6. Procédé selon l'une quelconque des revendications 1 à 5, **caractérisé en ce que** l'enveloppe de capsule contient en outre de l'eau, de préférence en une proportion de 50 à 60 % en poids, et/ou des polyols.

7. Procédé selon l'une quelconque des revendications précédentes, **caractérisé en ce que** l'enveloppe de capsule contient des lipides liquides à la température ambiante ou des mélanges liquides de ceux-ci et est solidifiée par incorporation de gouttelettes d'eau.

8. Procédé selon l'une quelconque des revendications précédentes, **caractérisé en ce que** l'enveloppe de capsule est constituée d'un mélange lipide-émulsifiant à stabilité dimensionnelle, qui contient de l'eau dispersée ayant une taille de gouttelette inférieure à 50 micromètres.

9. Procédé selon l'une quelconque des revendications précédentes, **caractérisé en ce que** les capsules ont un diamètre moyen de 3 à 40 mm.

10. Procédé selon l'une quelconque des revendications précédentes, **caractérisé en ce que** les capsules présentent des formes dans une large mesure sphériques, arrondies ou ellipsoïdes ayant un volume de 0,1 à 20 ml.

11. Procédé selon l'une quelconque des revendications précédentes, **caractérisé en ce que** l'enveloppe de capsule a une épaisseur de 0,001 à 3 mm, de préférence de 0,01 à 2 mm.

12. Procédé selon l'une quelconque des revendications précédentes, **caractérisé en ce que** le ou les composants du remplissage sont choisis dans le groupe constitué par des préparations aqueuses, des émulsions H/E, E/H, E/H/E, des gels, des hydrodispersions, des tensioactifs sous forme solide ou liquide, des microémulsions et/ou des mélanges de ceux-ci.

13. Procédé selon l'une quelconque des revendications précédentes, **caractérisé en ce que** le remplissage contient des substances lavantes et/ou des tensioactifs sous forme solide ou liquide et les composants de l'enveloppe comprennent des cires.
